(19) Europäisches Patentamt
European Patent Office
Office européen des brevets

(11) Numéro de publication: **0 428 421 A1**

(12) **DEMANDE DE BREVET EUROPEEN**

(21) Numéro de dépôt: **90402893.3**

(51) Int. Cl.[5]: **C07D 263/24**, A61K 31/42

(22) Date de dépôt: **16.10.90**

(30) Priorité: **17.10.89 FR 8913555**

(43) Date de publication de la demande: **22.05.91 Bulletin 91/21**

(84) Etats contractants désignés: **AT BE CH DE DK ES FR GB GR IT LI LU NL SE**

(71) Demandeur: **DELALANDE S.A.**
**32, rue Henri Regnault**
**F-92400 Courbevoie(FR)**

(72) Inventeur: **Jarreau, François-Xavier**
**5, rue Louis Hervé**
**F-78000 Versailles(FR)**
Inventeur: **Rovei, Vincenzo**
**44, rue Danton**
**F-92500 Rueil Malmaison(FR)**
Inventeur: **Koenig, Jean-Jacques**
**99, avenue du Général de Gaulle**
**F-78600 Maisons Laffitte(FR)**
Inventeur: **Schoofs, Alain**
**242, rue de la Croix-Nivert**
**F-75015 Paris(FR)**

(74) Mandataire: **Kedinger, Jean-Paul et al**
**c/o Cabinet Malemont 42, avenue du Président Wilson**
**F-75116 Paris(FR)**

(54) **Dérivés d'aryl-3 oxazolidinone, leur procédé de préparation et leur application en thérapeutique.**

(57) Dérivés répondant à la formule :

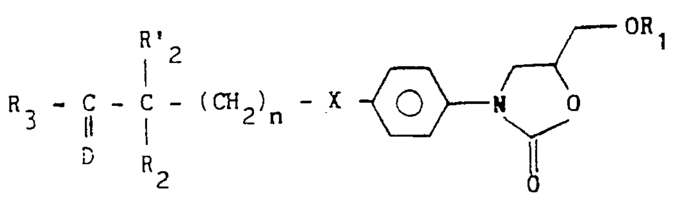

(I)

où :
- $R_1$ représente H ou alkyle en $C_1$-$C_4$ ;
- X représente un atome d'oxygène, un groupe méthylène ou un groupe -CH = CH- ;
- n prend la valeur 1 ou 2 quand X est un atome d'oxygène ou un groupe méthylène et la valeur 0 ou 1 quand X représente le groupe -CH = CH- ;
- D représente un atome d'oxygène ou un groupe NOR où R = H ou alkyle en $C_1$-$C_4$ ;
- $R_3$ représente un groupe alkyle en $C_1$-$C_4$,cycloalkyle en $C_3$-$C_7$, phényle ou benzyle ;
- $R_2$ et $R'_2$ représentent chacun indépendamment l'un de l'autre, un atome d'hydrogène ou un groupe alkyle en

EP 0 428 421 A1

$C_1$-$C_4$, cycloalkyle en $C_4$-$C_7$, phényle ou benzyle ; et
- $R'_2$ et $R_3$ peuvent en outre former ensemble une chaîne $-(CH_2)_3-$ ou $-(CH_2)_4-$,
utiles en tant que médicaments.

# DÉRIVÉS D'ARYL-3 OXAZOLIDINONE, LEUR PROCÉDÉ DE PRÉPARATION ET LEUR APPLICATION EN THÉRAPEUTIQUE

La présente invention a pour objet de nouveaux dérivés d'aryl-3 oxazolidinone-2, leur procédé de préparation et leur application en thérapeutique.

Ces dérivés répondent plus précisément à la formule :

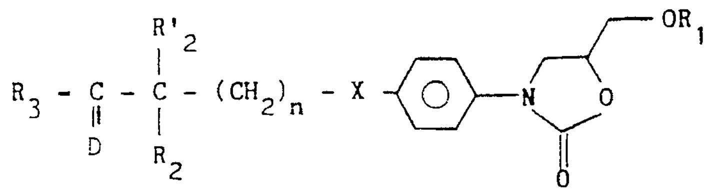

(I)

où :

- $R_1$ représente H ou alkyle en $C_1$-$C_4$ ;
- X représente un atome d'oxygène, un groupe méthylène ou un groupe -CH = CH- ;
- n prend la valeur 1 ou 2 quand X est un atome d'oxygène ou un groupe méthylène et la valeur 0 ou 1 quand X représente le groupe -CH = CH- ;
- D représente un atome d'oxygène ou un groupe NOR où R = H ou alkyle en $C_1$-$C_4$ ;
- $R_3$ représente un groupe alkyle en $C_1$-$C_4$,cycloalkyle en $C_3$-$C_7$, phényle ou benzyle ;
- $R_2$ et $R'_2$ représentent chacun indépendamment l'un de l'autre, un atome d'hydrogène ou un groupe alkyle en $C_1$-$C_4$, cycloalkyle en $C_4$-$C_7$, phényle ou benzyle ; et
- $R'_2$ et $R_3$ peuvent en outre former ensemble une chaine $-(CH_2)_3-$ ou $-(CH_2)_4-$.

Par ailleurs, il convient de souligner que les dérivés (I) comportent un ou plusieurs atomes de carbone asymétriques. Ils peuvent donc se présenter sous la forme de diastéréoisomères ou d'énantiomères ou sous forme cis ou trans ou encore sous la forme d'un mélange de toutes ces formes, y compris les formes racémiques. La présente invention s'étend par conséquent aux diverses formes ainsi définies, à l'exception des racémiques de formule (I) pour lesquels $R_1$ = $CH_3$, X = oxygène, $R_2$ = $R'_2$ = H, n = 1 ou 2 et D = oxygène.

La formule (I) ci-dessus couvre notamment les dérivés pour lesquels $R_1$ = H ou $CH_3$ ; X = oxygène ou $CH_2$ ; n = 1 ou 2 ; $R_2$ = $R'_2$ = H ou $CH_3$ ; $R_3$ = alkyle en $C_1$-$C_4$ ; et D représente oxygène, N-OH ou N-$OCH_3$.

On citera en particulier :

- les dérivés pour lesquels $R_1$ = $CH_3$ ; X = oxygène ; n = 1 ou 2 ; $R_2$ = $R'_2$ = H ; $R_3$ = $CH_3$ ; et D représente oxygène ;
- les dérivés pour lesquels $R_1$ = $CH_3$ ; X = oxygène ; n = 1 ou 2 ; $R_2$ = $R'_2$ = H ; $R_3$ = $CH_3$ ; et D représente N-OH (E) ;
- les dérivés pour lesquels $R_1$ = $CH_3$ ; X = méthylène ; n = 1 ou 2 ; $R_2$ = $R'_2$ = H ; $R_3$ = $CH_3$ ; et D représente oxygène ;
- les dérivés pour lesquels $R_1$ = $CH_3$ ; X = méthylène ; n = 1 ou 2 ; $R_2$ = $R'_2$ = H ; $R_3$ = $CH_3$ ; et D représente N-OH (E) ;
- les dérivés pour lesquels $R_1$ = $CH_3$ ; X représente CH = CH ; n = 0 ou 1 ; $R_2$ = $R'_2$ = H ; $R_3$ = $CH_3$ ; et D représente oxygène ; et
- les dérivés pour lesquels $R_1$ = $CH_3$ ; X représente CH = CH ; n = 0 ou 1 ; $R_2$ = $R'_2$ = H ; $R_3$ = $CH_3$ ; et D représente N-OH (E).

La présente invention a par ailleurs pour objet les procédés de préparation des dérivés (I).

Ces procédés sont essentiellement basés sur deux voies générales de synthèse.

La première de ces voies comprend l'élaboration d'une entité comportant le fragment oxazolidinone-2 (schémas 1 et 2), suivie du greffage sur cette entité d'une chaîne comportant un groupe précurseur de

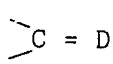

(schémas 3 à 6) et enfin transformation de ce groupe précurseur en

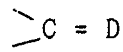

(schéma 7).

La deuxième de ces voies comprend, à l'inverse, d'abord l'élaboration de la chaîne comportant le groupe précurseur de

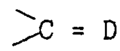

(schémas 8 à 11), suivie de l'élaboration et greffage sur cette chaîne d'une entité comportant le fragment oxazolidinone-2 (schémas 12 à 14) et enfin transformation du groupe précurseur en

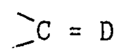

(schéma 7).

Ces quatorze schémas sont représentés ci-après. Sauf indication contraire, les symboles $R_1$, X, n, $R_2$, $R'_2$, $R_3$ et R apparaissant dans ces schémas, ont la même signification que dans la formule (I).

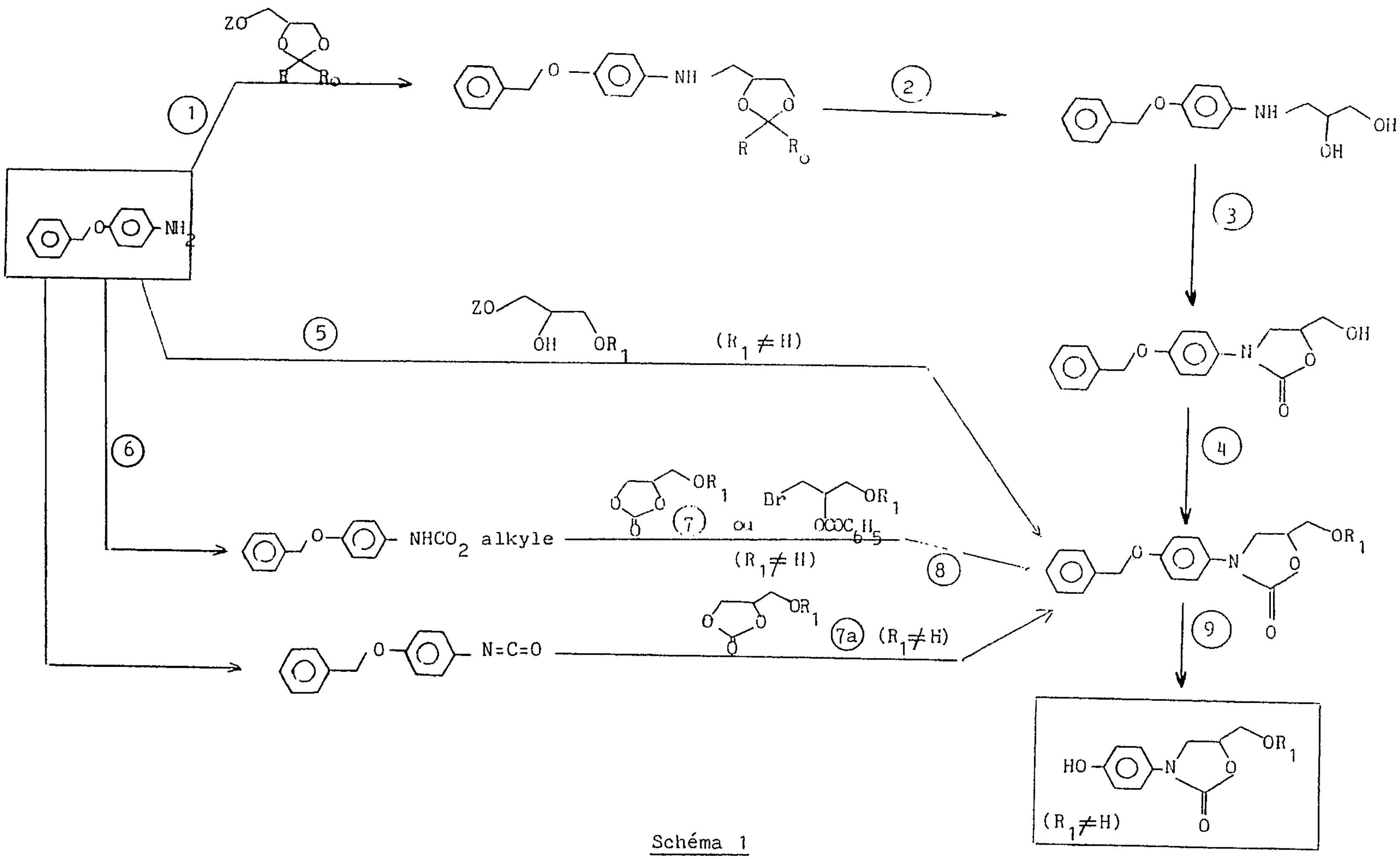


Schéma 1

. Z = Ms ou Ts

. R et $R_o$ représentent alkyle en $C_1$-$C_3$ ou forment ensemble $-(CH_2)_5-$

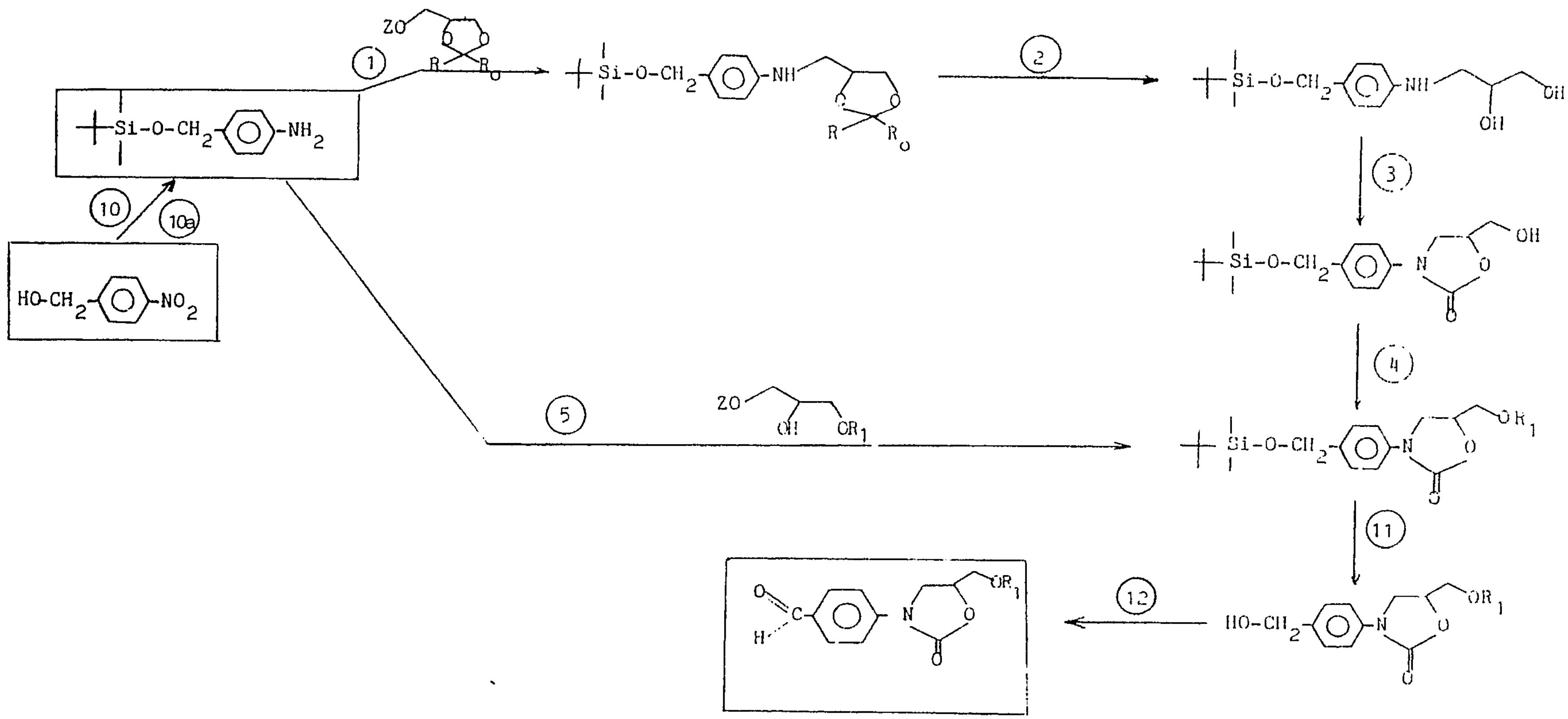

. $R_1 \neq H$

. Z = Ts ou Ms

. R et $R_o$ représentent alkyle en $C_1$-$C_3$ ou forment ensemble $-(CH_2)_5-$

Schéma 2

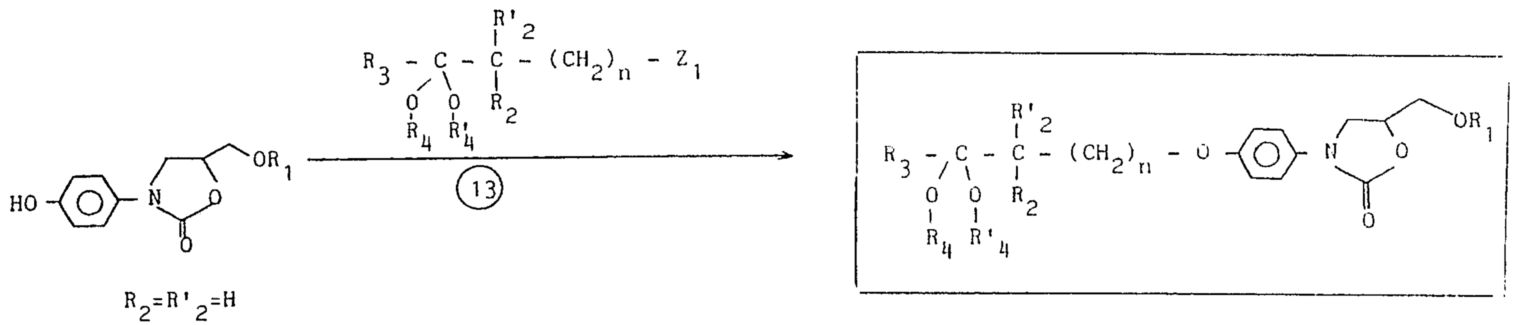

$R_2=R'_2=H$

$Z_1$ représente OTs, OMs ou halogène

Schéma 3

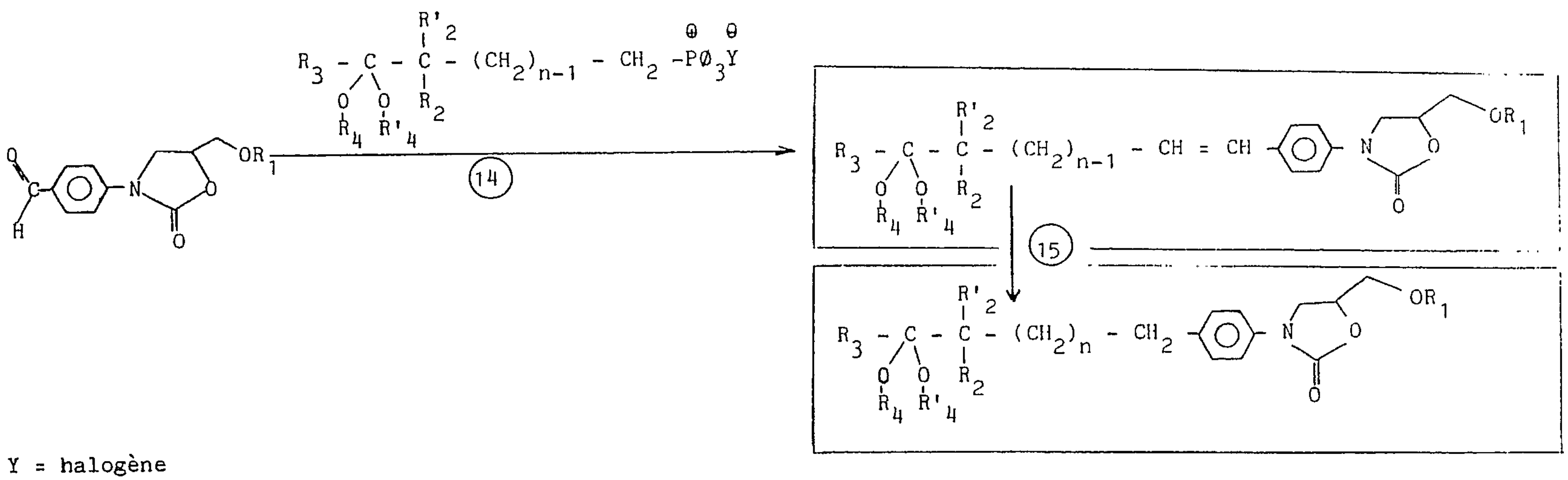

Y = halogène

$R_1 \neq H$

Schéma 4

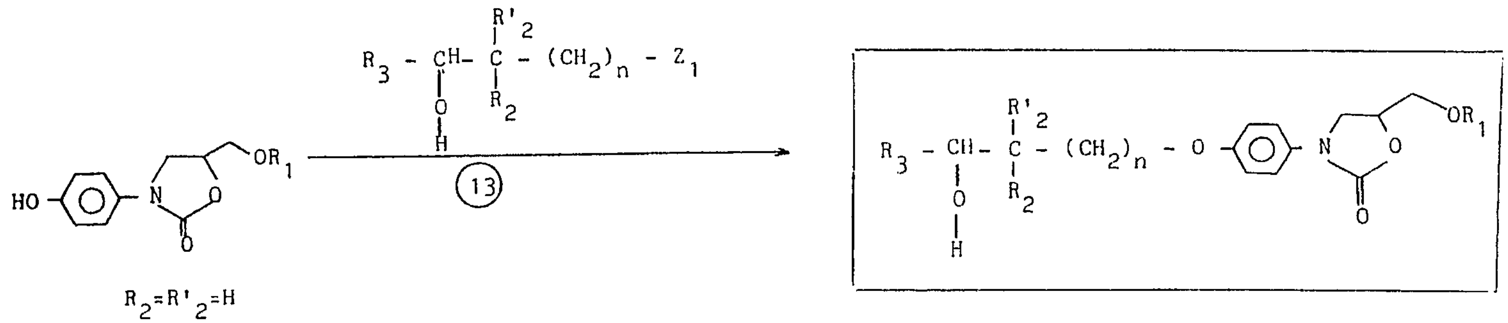

$R_2 = R'_2 = H$

$Z_1$ représente OTs, OMs ou halogène

Schéma 5

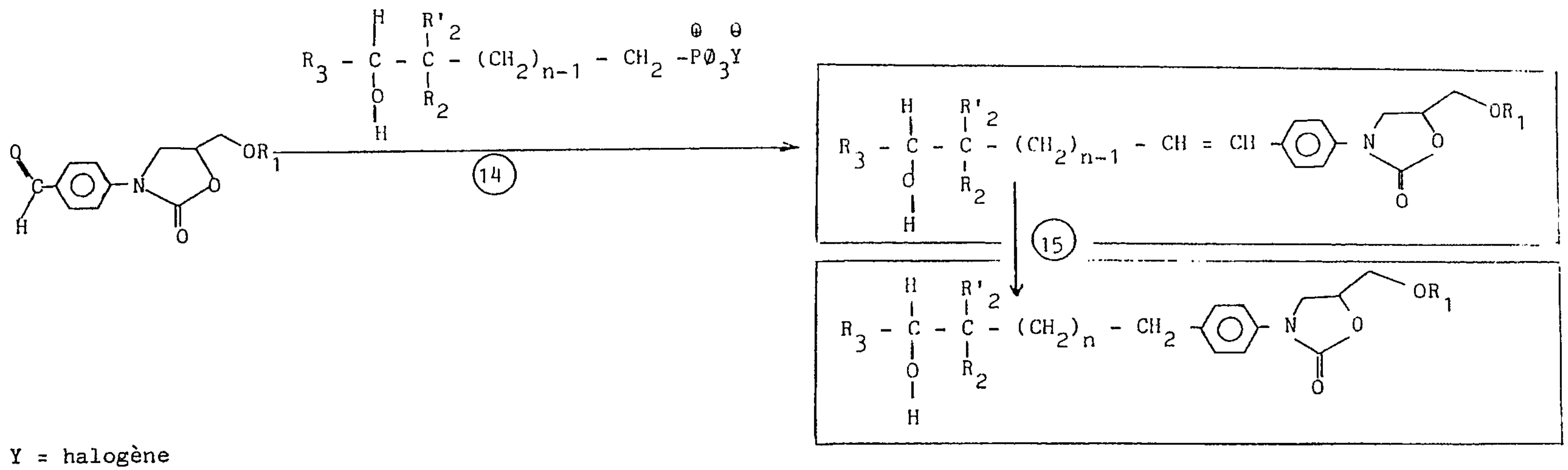

Y = halogène

$R_1 \neq H$

Schéma 6

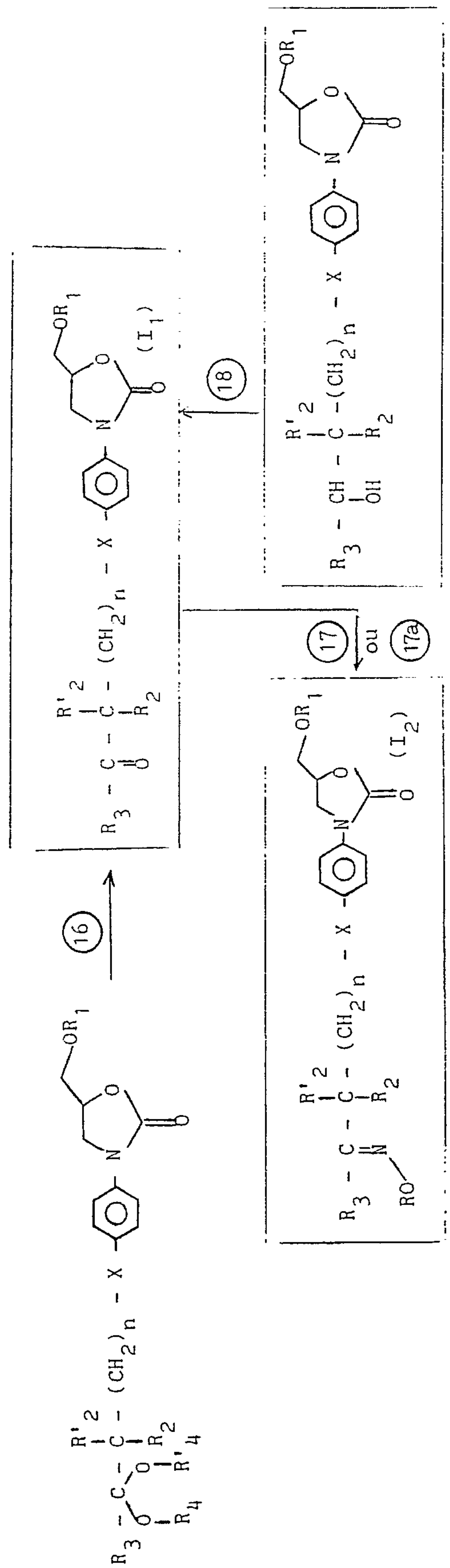


(I1)

(I2)

Schéma 7

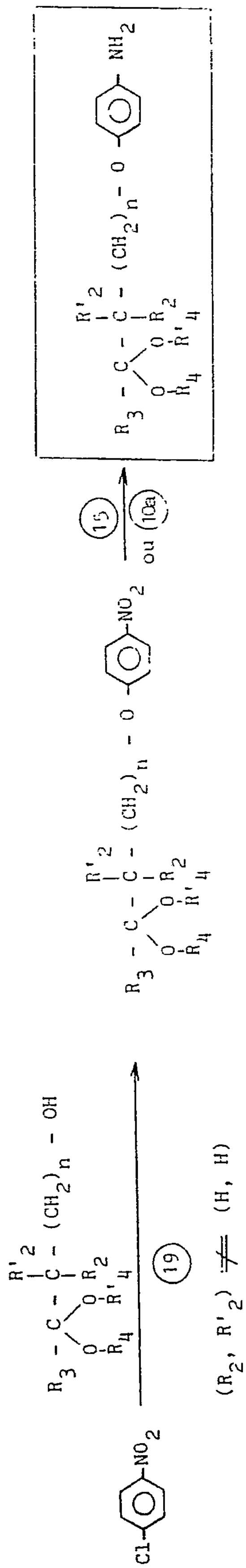


Schéma 8

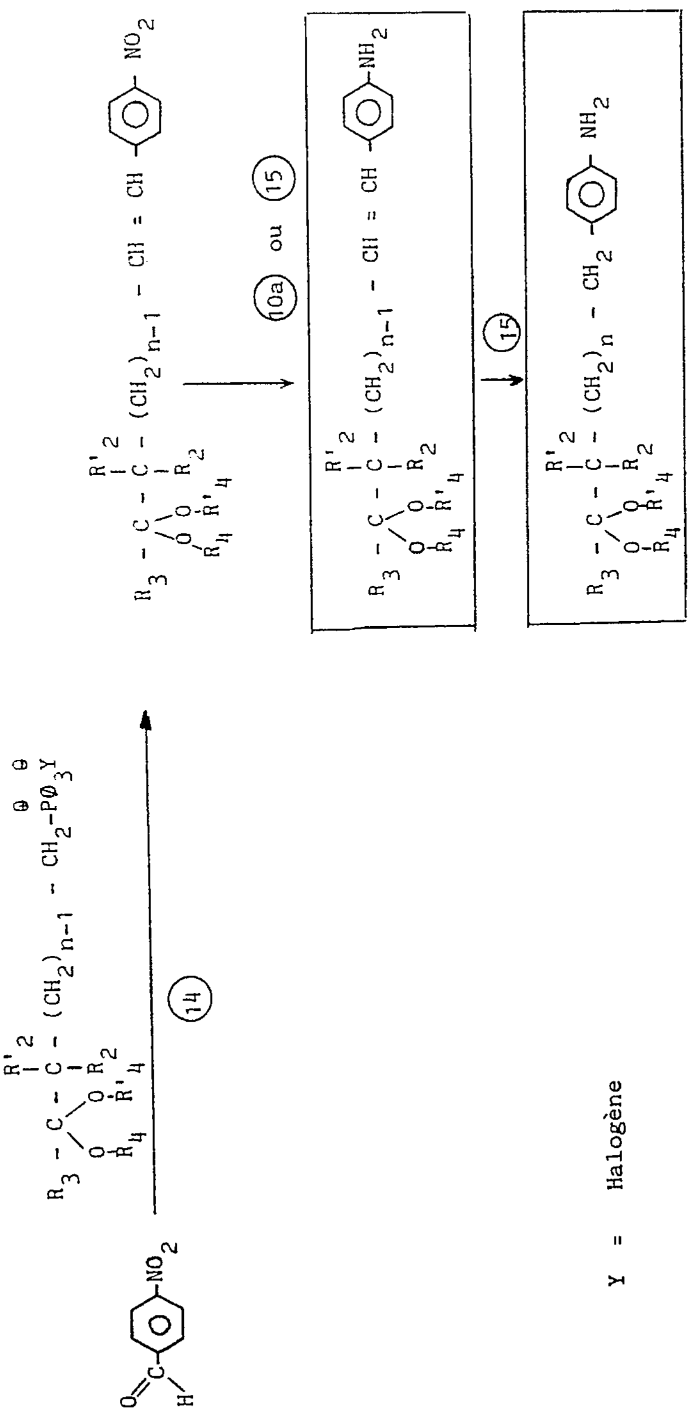

Schéma 9

Y = Halogène

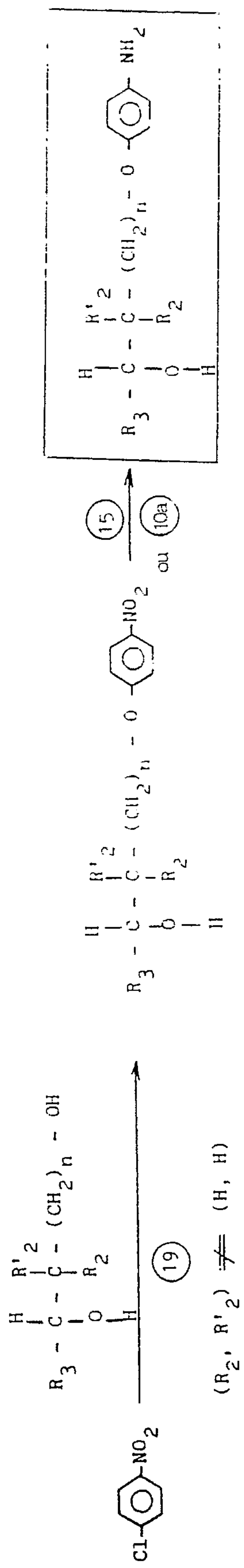


Schéma 10

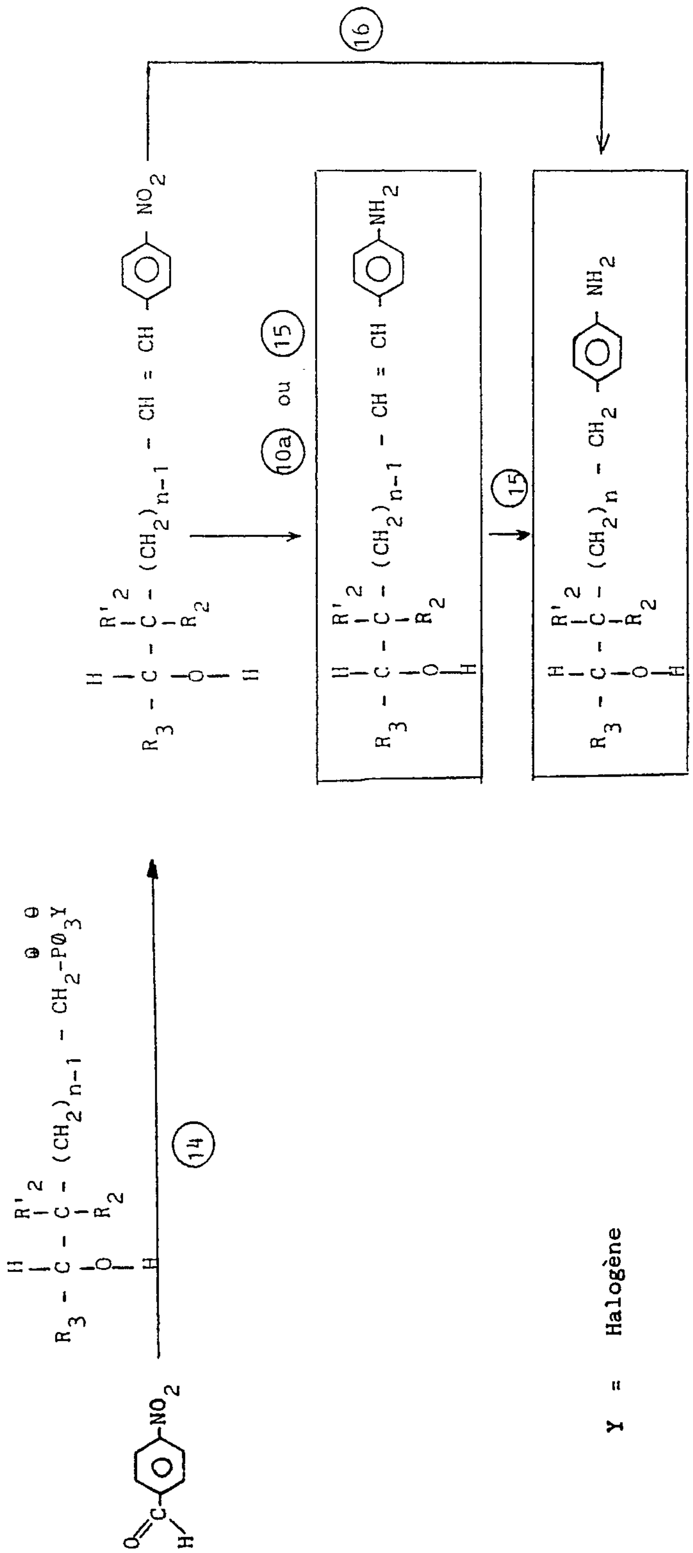


Schéma 11

Y = Halogène

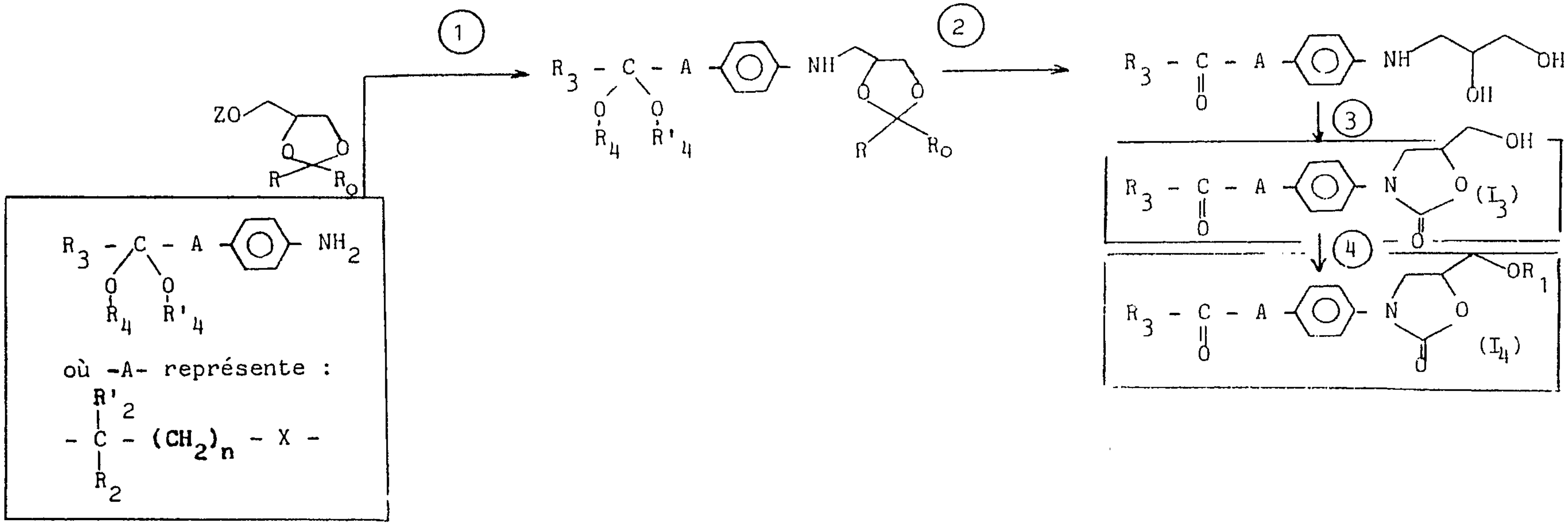


. Z = Ts ou Ms

. $R_1 \neq H$

. R et $R_o$ représentent alkyle en $C_1$-$C_4$ ou forment ensemble $-(CH_2)_5-$

Schéma 12

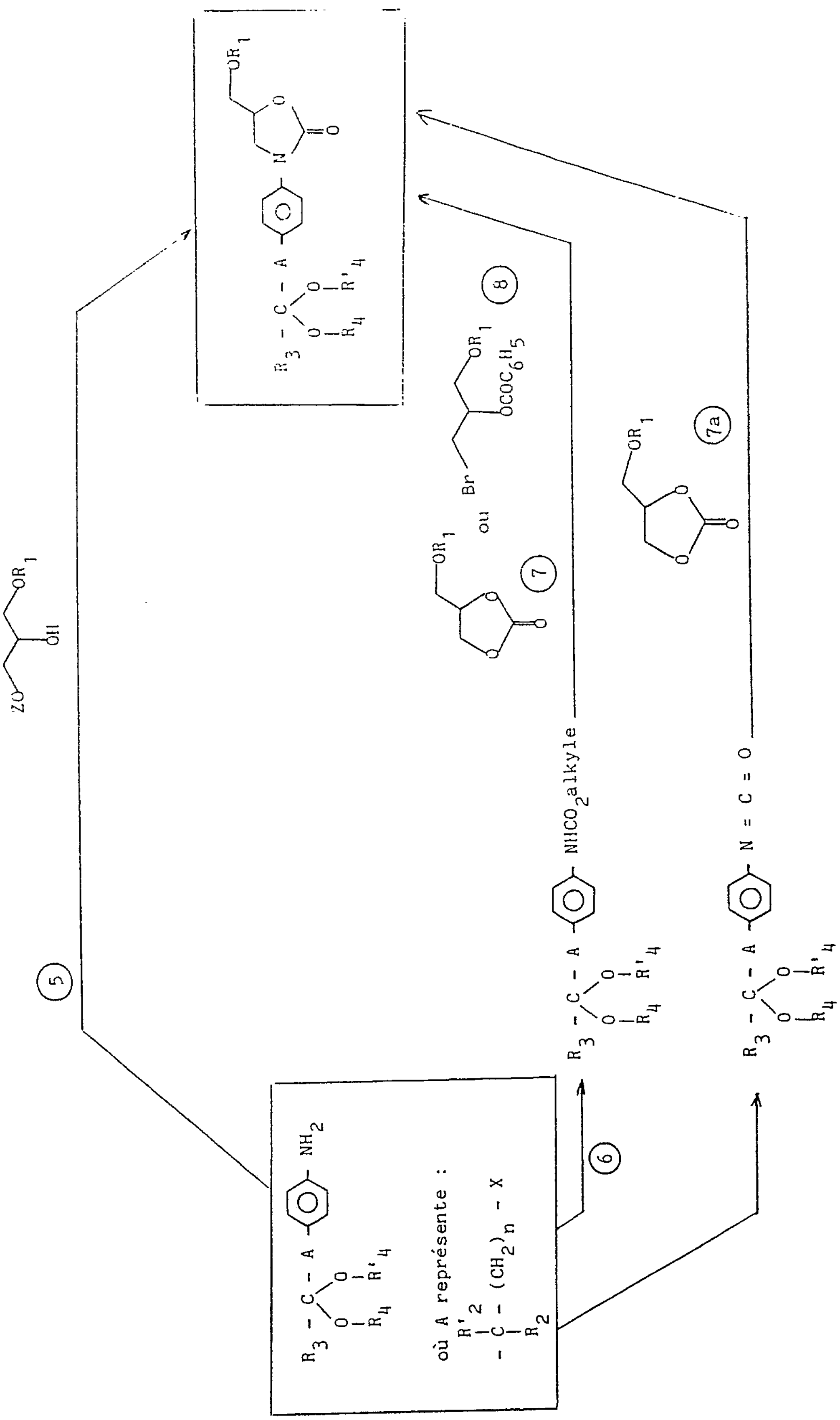


Schéma 13

$R_1 \neq H$

Z = Ts ou Ms

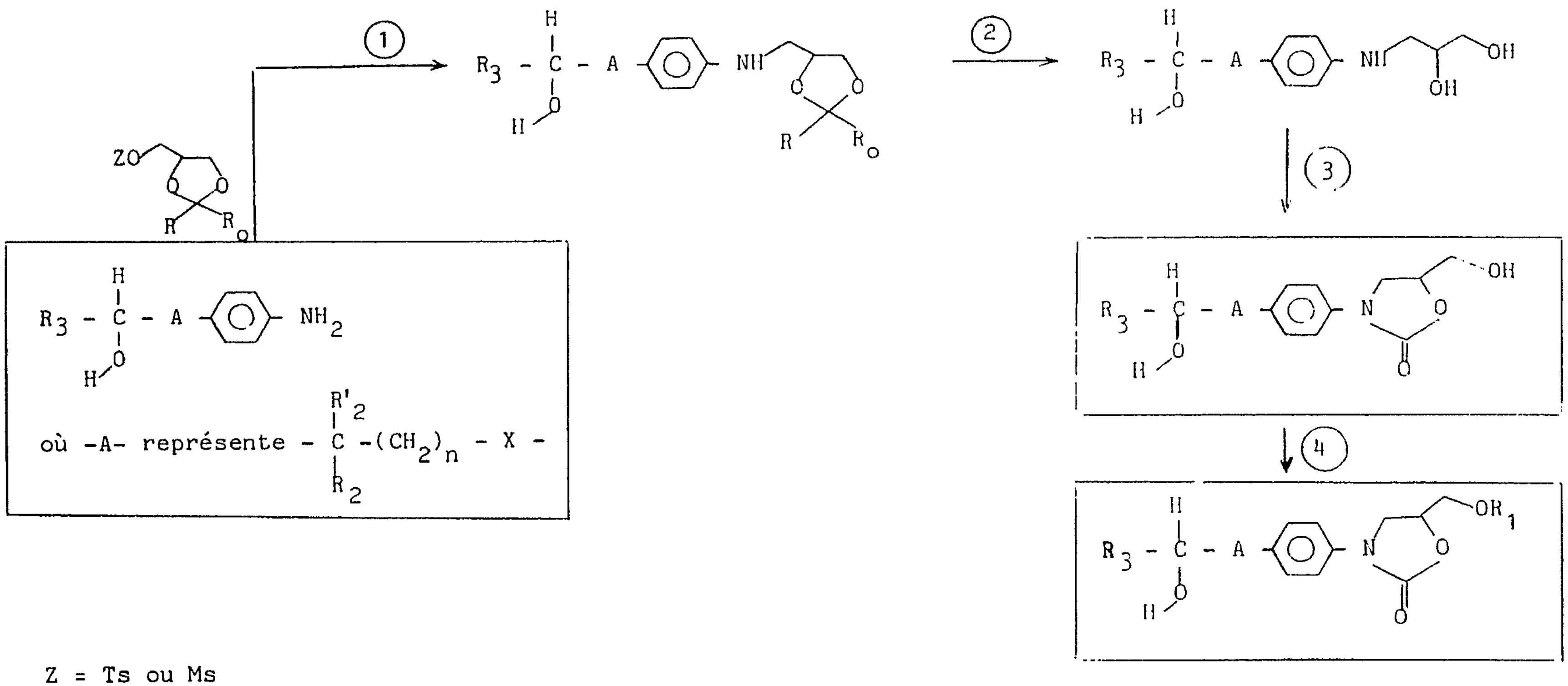


Z = Ts ou Ms

$R_1 \neq H$

R et $R_o$ représentent alkyle en $C_1$-$C_4$ ou forment ensemble une chaîne $-(CH_2)_5-$

Schéma 14

Par ailleurs, les composés de formules :

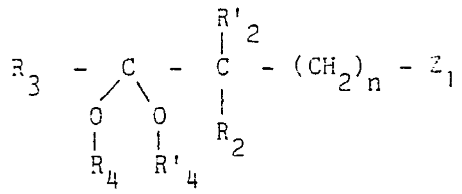

(schéma 3),

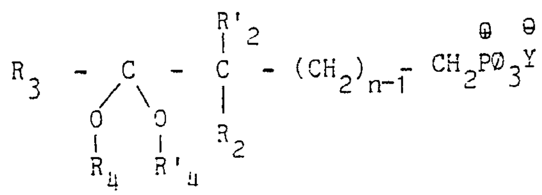

(schémas 4 et 9),

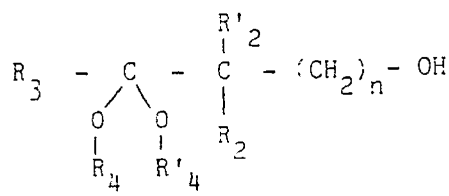

(schéma 8),

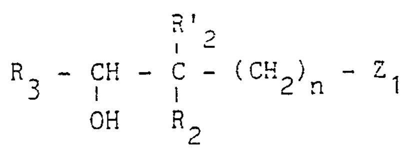

(schéma 5),

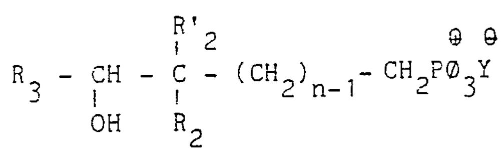

(schémas 6 et 11), et

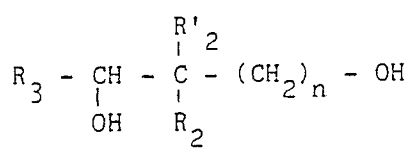

(schéma 10)

sont obtenus conformément aux schémas 15 et 16.

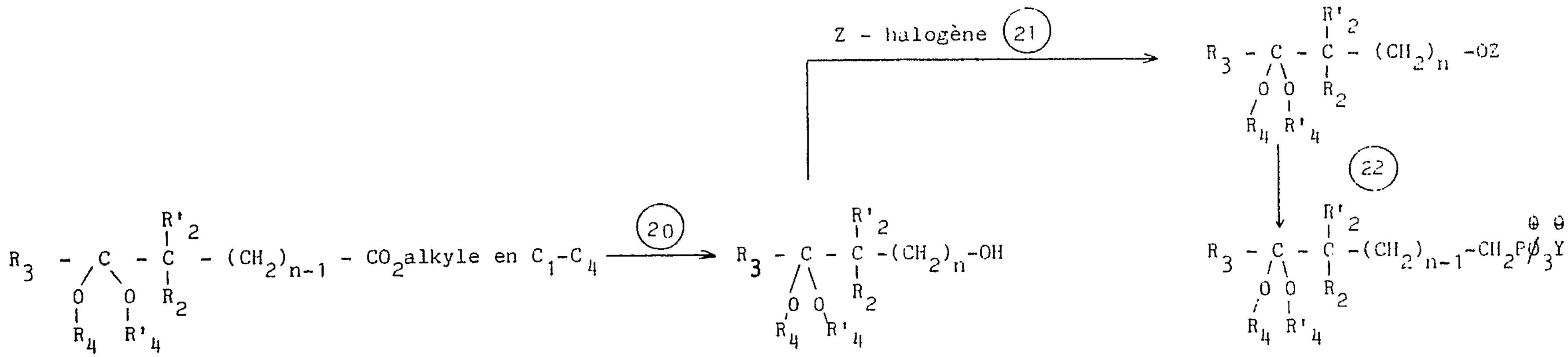


Z = Ts, Ms

Y = halogène

Schéma 15

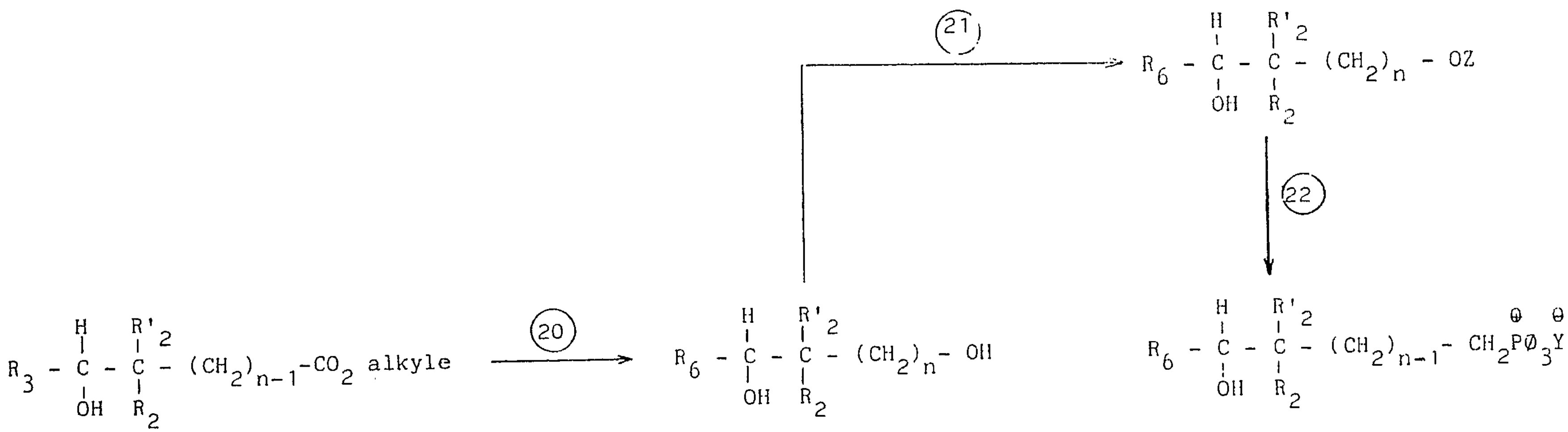


Z = Ts, Ms

Y = halogène

Schéma 16

Les indices

① à ㉒

apparaissant dans les schémas ci-dessus, ont les significations suivantes :

① Condensation soit dans un solvant aprotique anhydre comme le toluène, par chauffage, avec ou sans catalyseur comme le bromure d'hexadécyl tributyl phosphonium ou un halogénure d'ammonium quaternaire tel que le bromure de benzyl triéthylammonium, soit sans solvant en présence de triéthylamine entre 130-150° C.

② Hydrolyse par un acide aqueux notamment l'acide chlorhydrique 6N en présence d'un solvant organique comme la méthyléthylcétone.

③ Condensation avec un carbonate d'alkyle en $C_1$-$C_4$ notamment le carbonate de diéthyle dans un solvant anhydre comme le toluène en présence d'alcoolate de métal alcalin comme le méthylate de sodium.

④ Alcoylation par un halogénure (bromure ou chlorure) d'alkyle en $C_1$-$C_4$ dans les conditions de transfert de phase notamment soude-chlorure de méthylène ou toluène, en présence d'un ammonium quaternaire comme le bromure ou l'hydrogénosulfate de tétrabutylammonium.

⑤ Condensation en présence de phosgène et d'une base notamment la diméthylaniline dans un solvant organique comme le chlorure de méthylène, le dichloroéthane ou le toluène ; puis cyclisation par chauffage dans un solvant organique notamment alcoolique comme l'éthanol en présence d'une base notamment la potasse.

⑥ Condensation avec un chloroformiate d'alkyle, comme le chloroformiate d'éthyle, en présence d'une base notamment $NaHCO_3$ dans un mélange solvant eau-THF, à température ambiante.

⑦ Condensation à chaud (vers 150° C) en présence d'une base comme $K_2CO_3$. La réaction conserve la stéréochimie.

(7a)

Condensation dans le toluène en présence de LiBr et de $nBu_3PO$.

⑧ Condensation en présence d'une base notamment NaH dans un solvant aprotique comme le THF à 55° C-60° C.

⑨ Débenzylation dans un solvant alcoolique, comme le méthanol ou l'éthanol en présence d'hydrogène et d'un catalyseur notamment le palladium sur carbone à 10 % humidifié ou non.

⑩

O.silylation de l'alcool dans un solvant organique aprotique comme le THF ou DMF en présence d'une base notamment l'imidazole, et de terbutyldiméthylchlorosilane.

(10a)

Réduction du dérivé nitré par la poudre de fer en présence de chlorure d'ammonium.

⑪

Hydrolyse dans un solvant organique notamment le THF en présence de fluorure, notamment de tétrabutylammonium.

(12)

Oxydation en présence de chlorure d'oxalyle, de DMSO et d'une base, notamment la triéthylamine, dans un solvant organique aprotique comme le chlorure de méthylène.

(13)

O.alcoylation dans un solvant organique anhydre comme la méthyléthylcétone ou le DMF et en présence d'une base notamment $K_2CO_3$,

ou

O.alcoylation dans un solvant organique aprotique comme le DMF et/ou le THF et en présence d'un hydrure de métal alcalin comme l'hydrure de sodium.

(14)

Condensation en présence d'une base notamment $K_2CO_3$ et de formamide dans un solvant organique notamment le dioxanne, de préférence au reflux,

ou

Condensation en présence de LDA (lithium diisopropylamide) dans un mélange de solvants notamment DMSO/THF.

(15)

Hydrogénation sous pression atmosphérique d'hydrogène dans un solvant organique notamment l'acétate d'éthyle en présence d'un catalyseur comme le palladium sur carbone à 10 % humidifié ou non, ou $PtO_2$,

ou

Hydrogénation sous pression d'hydrogène notamment sous 5 atmosphères, en présence de palladium sur carbone à 10 % humidifié ou non, ou $PtO_2$, dans un solvant alcoolique, notamment l'éthanol,

ou

Hydrogénation sous pression d'hydrogène notamment sous 9 atmosphères, en présence de palladium sur carbone à 10 % humidifié ou non dans un solvant alcoolique, notamment l'éthanol.

(16)

Hydrolyse de préférence en présence de silice et de chlorure de fer hydraté dans un solvant organique notamment l'acétone ou la méthyléthylcétone.

(17)

Condensation avec un sel d'(alkoxy en $C_1$-$C_4$) amine ou d'hydroxylamine, de préférence dans un solvant alcoolique (par exemple EtOH) et en présence d'une base (par exemple $NaHCO_3$).

(17a)

Condensation avec l'hydroxylamine dans un solvant alcoolique, notamment l'éthanol.

(18)

Oxydation, par exemple par le dichromate de pyridinium de préférence en présence d'un acide comme l'acide acétique dans un solvant organique comme le chlorure de méthylène.

(19)

O.alcoylation dans un solvant organique aprotique notamment le DMF en présence d'hydrure de métal alcalin notamment le sodium.

(20)

Réduction dans un solvant organique aprotique comme le diméthoxyéthane en présence de borohydrure de lithium ou dans un solvant organique comme le THF en présence de $LiAlH_4$.

(21)

Condensation dans un solvant organique notamment la pyridine ou $CH_2Cl_2$ en présence d'une base notamment la diméthylamino-4 pyridine ou $Et_3N$.

(22)

Conformément à Helv. Chim. Acta 59, 755 (1976).

Il convient en outre de préciser que dans les schémas ci-dessus, $R_4$ et $R'_4$ représentent indépendamment l'un de l'autre alkyle en $C_1$-$C_4$ ou forment ensemble une chaîne $-(CH_2)_2$ ou $-(CH_2)_3$.

Les préparations suivantes sont données à titre d'exemples pour illustrer l'invention :

EXEMPLE 1:

Mélange racémique de diastéréoisomères d'[(hydroxy-3 butoxy)-4 phényl]-3 méthoxyméthyl-5 oxazolidinone-2 (numéro de code MD370047).

A une solution de 27,5 g (0,112 mole) de tosyloxy-1 butanol-3 dans 250 ml de méthyléthylcétone, on ajoute 28,2 g (0,2 mole) de $K_2CO_3$ et de 22,8 g (0,102 mole) d'(hydroxy-4 phényl)-3 méthoxyméthyl-5 oxazolidinone-2 (numéro de code MD780232). On chauffe au reflux pendant 4 h30. Après filtration et concentration, le résidu est repris dans 200 ml de $CH_2Cl_2$, la phase organique est lavée à l'eau saturée de NaCl, séchée sur $Na_2SO_4$ et concentrée. Après purification par flash chromatographique (silice, éluant : $CH_2Cl_2$ : 98 ; $CH_3OH$ : 2), on obtient le produit attendu avec un rendement de 70% ; F : 58 °C ; RMN$^1$H ($CDCl_3$) $\delta$ppm : 1,2 (3H) ; 1,8 (2H) ; 2,5 (1H éch.) ; 3,4 (3H) ; 3,6 (2H) ; 3,7-4,2 (5H) ; 4,7 (1H) ; 6,8 (2H) ; 7,4 (2H) ; IR (KBr) $\nu cm^{-1}$: 3400, 1750, 1730.

**Exemple 2 :**

[(hydroxy-3 (R) butoxy)-4 phényl]-3 méthoxyméthyl-5 (R) oxazolidinone-2 (numéro de code 370120)

Stade 1 : (benzyloxy-4 phenyl)amino-3 propane diol-1,2 (R) : (numéro de code MD 200418).

Dans un autoclave, on ajoute 1,5 kg de benzyloxy-4 aniline (7,564 moles), 0,2014 k de mésylate du dioxa-1,4 spiro [4,5] décane méthanol-2 (S) (8,048 moles) et 1,88 l de triéthylamine (13,5 moles). On chauffe les réactifs à 140 °C pendant 30 mn. Puis le milieu réactionnel est repris dans 7 l de méthyléthylcétone. La solution est lavée à l'eau et est utilisée pour l'étape suivante. A cette solution on ajoute 1,2 l d'acide chlorhydrique à 36 %. On chauffe le milieu réactionnel à 55 ° C pendant 30 mn et on refroidit à 20 ° C. On ajoute de la lessive de soude jusqu'à pH 9. La solution organique est lavée à l'eau et concentrée.

Le produit est obtenu avec un rendement de 90 % ; F : 102 °C ; $[\alpha]_D^{20}$ = + 12,7 ° (C = 1, $CH_3OH$).

Stade 2 :

(benzyloxy-4 phényl)-3 méthoxyméthyl-5 (R) oxazolidinone-2 (numéro de code MD 200404).

a) A une suspension de 13 g (0,0475 mole) du composé MD 200418 dans 100 ml de toluène, on ajoute au reflux 6,2 ml (0,052 mole) de carbonate d'éthyle et 2 ml de méthylate de sodium 1M dans le méthanol. On distille jusqu'à ce que le reflux atteigne le point d'ébullition du toluène. Après refroidissement, on ajoute du $CH_2Cl_2$ et la solution organique est lavée à l'eau et séchée sur $Na_2SO_4$. Après concentration, on obtient 14 g de (benzyloxy-4 phényl)-3 hydroxyméthyl-5 (R) oxazolidinone-2 : (numéro de code MD 220201) : F : 157 °C ; $[\alpha]_D^{20}$ : - 41 ° (C = 1, $CH_2Cl_2$).

b) A 15 g (0,05 mole) du produit précédent (MD 220201), on ajoute 100 ml de toluène et 18,9 g de sulfate de méthyle, 1,8 g de tétrabutyl ammonium hydrogénosulfate, 10 ml d'eau et 10 g de NaOH. On chauffe les réactifs 1/2 h. Le milieu réactionnel est extrait à l'éther isopropylique et le produit attendu est obtenu avec un rendement de 83 % ; F : 101 °C ; $[\alpha]_D^{20}$ : - 41,5 ° (C = 1, $CH_2Cl_2$).Par un mode opératoire identique mais à partir des réactifs appropriés on a obtenu le (benzyloxy-4 phényl)-3 méthoxyméthyl-5 (S) oxazolidinone-2 (numéro de code 340190), F : 101 °C , $[\alpha]_D^{20}$ : + 41,9 ° (C = 1, $CH_2Cl_2$) ; ainsi que (benzyloxy-4 phényl)-3 éthoxyméthyl-5 (R) oxazolidinone-2 (numéro de code MD 230242), F : 78 ° C, $[\alpha]_D^{20}$ : - 35,9 ° (C = 1, $CH_2Cl_2$) ; IR (KBr)$\nu$ $cm^{-1}$ : 1750, 1735.

Stade 3 :

(Hydroxy-4 phényl)-3 méthoxyéthyl-5 (R) oxazolidinone-2 (numéro de code MD 200405).

A une solution de 13 g (0,047 mole) de composé MD 200404 dans 80 ml d'éthanol et 40 ml de $CH_2Cl_2$ en présence de 2,6 g de Pd/C à 10 % humidifié à 50 %, on fait passer un courant d'hydrogène sous pression normale.

Lorsque la réaction est complète, la solution est filtrée et concentrée. Le produit attendu est obtenu avec un rendement de 100 %. F : 112 °C ; $[\alpha]_D^{20}$ : - 67 ° (C = 1, $CH_3OH$) ; IR (KBr) $\nu cm^{-1}$ : 3260, 1730.

Par un procédé identique mais à partir des réactifs correspondants, on obtient le dérivé (hydroxy-4 phényl)-3 méthoxyméthyl-5 (S) oxazolidinone-2 : (numéro de code MD 200717) : F : 114 °C, $[\alpha]_D^{20}$ : + 66 ° (C = 1, $CH_3OH$) ; ainsi que le dérivé (hydroxy-4 phenyl)-3 éthoxyméthyl-5 (R) oxazolidinone-2 (numéro de code MD 230243), F : 92 ° C ; $[\alpha]_D^{20}$ : - 58,9 ° (C = 1, $CH_3OH$), IR (KBr)$\nu$ $cm^{-1}$ : 3300, 1770.

Stade 4 :

[(hydroxy-3 (R) butoxy)-4 phényl]-3 méthoxyméthyl-5 (R) oxazolidinone-2 : (numéro de code MD 370120).

A 100 ml de méthyléthylcétone, on ajoute 14,6 g (0,059 mole) de tosyloxy-1 butanol-3 (R) (Helv. Chim. Acta, 67, 89, 1984), 14,8 g (0,1 mole) de $K_2CO_3$ et 18 g (0,053 mole) de composé MD 200405. On chauffe au reflux 5 h, après filtration, le milieu réactionnel est concentré, repris dans l'acétate d'éthyle, lavé à l'eau, séché sur $Na_2SO_4$ et concentré. Le produit est purifié par chromatographie flash (silice, éluant : $CH_2Cl_2$ : 95 ; $CH_3OH$ : 5) ; F : 76 °C $[\alpha]_D^{20}$ : - 50,7 ° (C = 1, $CH_2Cl_2$).Par un mode opératoire identique en faisant réagir le tosylate-1-butanol-3 (R) avec le MD 200717 on obtient l'[hydroxy-3 (R) butoxy)-4 phényl]-3 méthoxyméthyl-5 (S) oxazolidinone-2 (numéro de code MD 370123) : F : 44 °C,$[\alpha]_D^{20}$ : + 33 ° (C = 1, $CH_2Cl_2$).

De même, en faisant réagir le tosyloxy-1 butanol-3 (S) (J. Org. Chem. 47, 3850, 1982) avec le composé MD 200405 on obtient l'[(hydroxy-3 (S) butoxy)-4 phényl]-3 méthoxyméthyl-5 (R) oxazolidinone-2 (numéro de code MD 370122), et avec le composé MD 200717 on obtient l'[(hydroxy-3 (S) butoxy)-4 phényl]-3 méthoxyméthyl-5 (S) oxazolidinone-2 (numéro de code MD 370121).

EXEMPLE 3 :

[(Méthyl-2 dioxolane-1,3 yl-2)-2 éthoxy]-4 phényl]-3 méthoxyméthyl-5 (R) oxazolidinone-2 (numéro de code MD370296).

A une solution de 50 ml de DMF, on ajoute 3 g ((0,076 mole) de NaH à 60 % et en 15 mn, 16 g (0,076 mole) du composé MD200405 (ex. 2, stade 3) en solution dans 75 ml de DMF. Puis en maintenant la température à 20 °C, on ajoute 0,836 mole de (mésyloxy-2 éthyl)-2 méthyl-2 dioxolane en solution dans 25 ml de DMF. Le milieu réactionnel est laissé 24 heures à température ambiante et versé sur l'eau glacée. La phase aqueuse est extraite au $CH_2Cl_2$ et la phase organique est séchée au sulfate de magnésium. Le produit est obtenu après purification sur colonne de silice (éluant : heptane : 40 ; acétate d'éthyle : 60) avec un rendement de 44 % ; F : 48 °C ; $[\alpha]_D^{20}$ : - 32,8° (C = 1, $CH_2Cl_2$) ; RMN$^1$H ($CDCl_3$) δppm : 1,4 (3H) ; 2,2 (2H) ; 3,4 (3H) ; 3,6 (2H) ; 3,9 (4H) ; 3,7-4,3 (4H), 4,7 (1H) ; 6,8 (2H) ; 7,4 (2H) ; IR (KBr) $\nu cm^{-1}$ : 1740 ; RMN$^{13}$C : Cq : 155,6 ; 154,4 ; 131,5 ; 108,7 ; CH : 120,2 ; 114,9 ; 71,2 ; $CH_2$ : 72,7 ; 64,6 ; 64,3 ; 47,5 ; 38,2 ; $CH_3$ : 59,6; 24,4.

De la même manière ont été obtenues :

[(Méthyl-2 dioxolane-1,3 yl-2)-3 propoxy]-4 phényl]-3 méthoxyméthyl-5 (R) oxazolidinone-2 (numéro de code MD370506) : RMN$^1$H ($CDCl_3$) δppm : 1,35 (3H) ; 1,8 (4H) ; 3,4 (3H) ; 3,6 (2H) ; 3,7-4,2 (4H) , 3,9 (4H) ; 4,7 (1H) ; 6,8 (2H) ; 7,4 (2H) ; IR (KBr) $\nu cm^{-1}$ : 1750 ; F : 67 °C.

[(Méthyl-2 dioxolane-1,3 yl-2)-2 éthoxy]-4 phényl]-3 hydroxyméthyl-5 (R) oxazolidinone-2 (numéro de code MD230046) : RMN$^1$H ($CDCl_3$) δppm : 1,4 (3H) ; 2,15 (2H) ; 3 (1H éch.) ; 3,9 (4H) ; 3,6-4,2 (6H ) ; 4,6 (1H) ; 6,2 (2H) ; 7,4 (2H) ; IR (KBr) $\nu cm^{-1}$ : 3480, 1710 ; $[\alpha]_D^{20}$ : - 40,2 ° (C = 1, $CH_2Cl_2$);F : 132 °C ; rendement : 96 %.

(Dioxa-1,4 spiro[4,4] nonane [1,4]-yl-6 méthoxy)-4 phényl]-3 méthoxyméthyl-5 (R) oxazolidinone-2 (numéro de code MD230204) : $[\alpha]_D^{20}$: - 44,8 ° (C = 1, $CH_3OH$); RMN$^1$H ($CDCl_3$) δppm : 1,4-2,6 (7H) ; 3,4 (3H) ; 3,6 (2H) ; 3,8-4,2 (8H) ; 4,7 (1H) ; 6,9 (2H) ; 7,4 (2H) ; huile.

EXEMPLE 4:

[(Oxo-3 butoxy)-4 phényl]-3 méthoxyméthyl-5 (R) oxazolidinone-2 (numéro de code MD370268).

*Méthode 1*

A une solution de 1,9 g (5,07 $10^{-3}$ moles) de dichromate de pyridinium dans 15 ml de chlorure de méthylène, on ajoute une goutte d'acide acétique et du tamis moléculaire 4Å . Puis à 0 °C, on ajoute une solution de 1 g du composé MD370120 (ex. 2) (3,38 $10^{-3}$ moles) dans 5 ml de chlorure de méthylène. Après 30 mn à 0 °C, on laisse revenir à température ambiante et on ajoute 5 g de célite. Après filtration et concentration, le résidu est repris dans l'éther éthylique. La phase organique est concentrée et le précipité est organisé dans un mélange éther isopropylique/éther éthylique (80/20). Le produit attendu est obtenu avec un rendement de 80 % ; F : 49 °C ; $[\alpha]_D^{20}$ : - 42,6 ° (C = 1, $CH_2Cl_2$) ; RMN$^1$H ($CDCl_3$) δppm : 2,2 (3H) ; 2,85 (2H) ; 3,4 (3H) ; 3,6 (2H) ; 3,8-4,4 (4H) ; 4,7 (1H) ; 6,8 (2H) ; 7,4 (2H) ; IR (KBr) $\nu cm^{-1}$ : 1750, 1710.

*Méthode 2*

A une solution de 294 g (0,871 mole) du composé MD370296 (exemple 3) dans 2,5 l d'acétone, on ajoute 600 g de ($FeCl_3$, $6H_2O$, $SiO_2$)n en 10 mn. Après 4 heures d'agitation, le milieu réactionnel est filtré et

séché sur $Na_2SO_4$ et concentré. Le produit est obtenu avec 74,1 % de rendement en ayant les mêmes caractéristiques que par la méthode 1.

Par une des deux méthodes décrites précédemment ont également été obtenues :

[(Oxo-4 pentoxy)-4 phényl]-3 méthoxyméthyl-5 (R) oxazolidinone-2 (numéro de code MD370507) : RMN$^1$H ($CDCl_3$) δppm : 2 (2H) ; 2,15 (3H) ; 2,6 (2H) ; 3,4 (3H) ; 3,6 (2H) ; 3,9 (4H) ; 4,65 (1H) ; 6,8 (2H), 7,4 (2H) ; IR (KBr) νcm$^{-1}$ : 1760, 1710 ; $[\alpha]_D^{20}$ : - 40,3 ° (C = 1, $CH_2Cl_2$) ; F : 70 °C.

[(Oxo-3 butoxy)-4 phényl]-3 hydroxyméthyl-5 (R) oxazolidinone-2 (méthode 1) (numéro de code MD230047) : RMN$^1$H ($CDCl_3$) δppm : 2,2 (3H) ; 2,9 (2H) ; 3,3-4,3 (4H) ; 4,2 (2H) ; 4,7 (1H) ; 5,2 (1H éch.) ; 6,9 (2H) ; 7,5 (2H) ; IR (KBr) νcm$^{-1}$ : 3450, 1720 ; $[\alpha]_D^{20}$ : - 49,4 ° (C = 1, $CH_3OH$) ; F : 126 °C.

[(Oxo-1 cyclopentyl-2 méthoxy)-4 phényl]-3 méthoxy méthyl-5 (R) oxazolidinone-2 (numéro de code MD230200) ; F : 70 °C ; $[\alpha]_D^{20}$ : - 51,2 ° (C = 1, $CH_3OH$);RMN$^1$H ($CDCl_3$) δppm : 1,8-2,6 (7H) ; 3,4 (3H) ; 3,66 (2H) ; 3,8-4,2 (4H) ; 4,7 (1H) ; 6,9 (2H) ; 7,4 (2H).

EXEMPLE 5:

[(N-hydroxy imino-3 butoxy)-4 phényl]-3 méthoxyméthyl (R)-5 oxazolidinone-2 (mélange E et Z) (numéro de code MD370298).

A une solution de 3,5 g (0,05 m) de chlorhydrate d'hydroxylamine et de 4,2 g (0,05 mole) de bicarbonate de sodium dans 24 ml d'eau, on ajoute une solution de 1,2 g (0,04 mole) de composé MD370268 (exemple 4) dans 80 ml d'éthanol. Après 30 mn d'agitation, le milieu réactionnel est concentré et repris dans un mélange eau et $CH_2Cl_2$. La phase organique est séchée sur $Na_2SO_4$ et concentrée. Le produit après recristallisation dans l'éthanol est obtenu avec un rendement de 73 % ; F : 105 °C ; $[\alpha]_D^{20}$ : - 40,3 ° (C = 1, $CH_2Cl_2$) ; RMN$^1$H (DMSO $d_6$) δppm : 1,85 (3H) ; 2,7 (2H) ; 3,35 (3H) ; 3,6 (2H) ; 3,7-4,3 (4H) ; 4,8 (1H) ; 6,9 (2H) ; 7,4 (2H) ; 10,3 (1H éch.) ; IR (KBr) νcm$^{-1}$ : 3430, 1730.

De la même manière ont été obtenues :

[(N-hydroxy imino-3 butoxy)-4 phényl]-3 méthoxyméthyl-5 oxazolidinone-2 (numéro de code MD370307) : RMN$^1$H (DMSO $d_6$) δppm : 1,8 et 1,85 (3H) ; 2,7 (2H) ; 3,35 (3H) ; 3,6 (2H) ; 3,7-4,3 (4H) ; 4,8 (1H) ; 6,9 (2H) ; 7,4 (2H) ; 10,3 (1H éch.) ; IR (KBr) νcm$^{-1}$ : 3350, 1740;

[(N-méthoxy imino-3 butoxy)-4 phényl]-3 méthoxyméthyl-5 oxazolidinone-2 (numéro de code MD370326) : RMN$^1$H ($CDCl_3$) δppm : 1,85 et 1,90 (3H) ; 2,7 (2H) ; 3,4 (3H) ; 3,6 (2H) ; 3,8 (3H) ; 3,8-4,3 (4H) ; 4,7 (1H) ; 6,8 (2H) ; 7,4 (2H) ; IR (microcellule) νcm$^{-1}$ : 1750;

[(N-méthoxy imino-3 butoxy)-4 phényl]-3 méthoxyméthyl-5 (R) oxazolidinone-2 : (numéro de code MD370299) : IR (microcellule) νcm$^{-1}$ : 1760, 1730 ; $[\alpha]_D^{20}$ : - 39,4 ° (C = 1, $CH_2Cl_2$) ; RMN ($CDCl_3$) δppm : 1,85 et 1,90 (3H) ; 2,7 (2H) ; 3,4 (3H) ; 3,6 (2H) ; 3,8 (3H) ; 3,7-4,3 (4H) ; 4,7 (1H) ; 6,8 (2H) ; 7,4 (2H) ;

[(N-hydroxy imino-4 pentoxy)-4 phényl]-3 méthoxyméthyl-5 (R) oxazolidinone-2 : (numéro de code MD370508) : RMN$^1$H ($CDCl_3$) δppm : 1,9 (3H) ; 1,8-2,7 (4H) ; 3,4 (3H) ; 3,6 (2H) ; 3,7-4,2 (4H) ; 4,7 (1H) ; 6,9 (2H) ; 7,4 (2H) ; 9,1 (1H éch.) ; IR (KBr) νcm$^{-1}$ : 3460, 1740, 1720 ; $[\alpha]_D^{20}$ : - 39 ° (C = 1, $CH_2Cl_2$) ; F : 83 °C;

[(N-hydroxy imino-3 butoxy)-4 phényl]-3 hydroxyméthyl-5 (R) oxazolidinone-2 (numéro de code MD230001) : RMN$^1$H (DMSO $d_6$) δppm : 1,90 et 1,95 (3H) ; 2,7 (2H) ; 3,6-4,4 (6H) ; 4,8 (1H) ; 5,3 (1H éch.) ; 7 (2H) ; 7,4 (2H) ; 10,5 (1H éch.) ; IR (KBr) νcm$^{-1}$ : 3450, 1730, 1700 ; $[\alpha]_D^{20}$ : - 47,4 ° (C = 1, $CH_3OH$) ; F : 147,5 °C.

EXEMPLE 6:

[(N-hydroxy imino-3 (E) butoxy)-4 phényl]-3 méthoxyméthyl-5 (R) oxazolidinone-2 (E) (numéro de code MD230050).

A une solution de 24 g (8,18 $10^{-2}$ moles) du composé MD370268 (exemple 4) dans 240 ml d'éthanol à 96, on ajoute 9,1 g (0,13 mole) de chlorhydrate d'hydroxylamine en 40 mn. Après 1 h 30 d'agitation, le produit attendu est filtré et obtenu avec un rendement de 80 % ; RMN$^1$H ($CDCl_3$) δppm : 1,8 (3H) ; 2,6 (2H) ; 3,3 (3H) ; 3,6 (2H) ; 3,6-4,4 (4H) ; 4,8 (1H) ; 6,9 (2H) ; 7,5 (2H) ; 10,2 (1H éch.) ; IR (KBr) νcm$^{-1}$ : 3420, 1730 ; $[\alpha]_D^{20}$ : - 41,4 ° (C = 1, $CH_2Cl_2$) ; F : 119 °C.

EXEMPLE 7 :

[(N-hydroxy imino-3 (Z) butoxy)-4 phényl]-3 méthoxyméthyl -5 (R) oxazolidinone-2 (numéro de code MD230062) : a été obtenue par chromatographie (silice, éluant : $CH_2Cl_2$ : 97 ; isopropanol : 3) du mélange E et Z MD 370298 (exemple5) ; F : 114 °C ; RMN[1]H (DMSO $d_6$) δppm : 1,85 (3H) ; 2,7 (2H) ; 3,3 (3H) ; 3,55 (2H) ; 3,6-4,25 (2H) ; 4,15 (1H) ; 4,6-5 (1H) ; 6,9 (2H) ; 7,5 (2H) ; 10,4 (1H) ; IR (KBr) $\nu cm^{-1}$ : 3240, 1730.

EXEMPLE 8 :

[(Oxo-3 diméthyl-2,2 butoxy)-4 phényl]-3 hydroxyméthyl-5 (R) oxazolidinone-2 (numéro de code MD230109).

Stade 1 :

Diméthyl-2,2 (méthyl-2 dioxolan-1,3-yl)-2 éthanol (numéro de code MD230103).

Une solution de 35 g (0,073 mole) d'ester éthylique de l'acide diméthyl-2,2 (méthyl-2 dioxolane-1,3 yl-2)- 2 acétique dans 50 ml de THF est ajoutée à 0 °C à une suspension de 7,23 g (0,19 mole) de $LiAlH_4$ dans 300 ml de THF en 15mn. Puis le milieu réactionnel est hydrolysé par 20 ml d'eau. Après filtration et concentration, le produit est obtenu avec un rendement de 92 % ; IR (KBr) $\nu cm^{-1}$ : 3450, 2980, 2880 ; RMN[1]H ($CDCl_3$) δ ppm : 1 (6H) ; 1,2 (3H) ; 3,5 (2H) ; 4 (4H).

Stade 2 :

Méthyl-2 [(nitro-4 phénoxy)-2 diméthyl-1,1 éthyl]-2 dioxolane-1,3 (numéro de code MD230105).

A une solution de 1,6 g (0,01 mole) de MD230103 dans 13 ml de DMF, on ajoute 0,48 g (0,01 mole) de NaH à 50 %. Après 15 mn d'agitation, on ajoute une solution de 1,32 g (0,0084 mole) de parachloronitrobenzène et agite à température ambiante 30 mn.

Le milieu réactionnel est versé sur l'eau et extrait à l'éther isopropylique. Les phases organiques sont lavées à l'eau saturée de NaCl, séchées sur $Na_2SO_4$, concentrées. Le produit est purifié par chromatographie flash (silice, éluant : heptane : 80 ; acétate d'éthyle : 20) ; rendement : 68 % ; huile ; RMN[1]H ($CDCl_3$) δppm : 1,1 (6H) ; 1,3 (3H) ; 4 (6H) ; 6,9 (2H) ; 8,1 (2H).

Stade 3 :

Méthyl-2 [(amino-4 phénoxy)-2 diméthyl-1,1 éthyl]-2 dioxolane-1,3 (numéro de code MD230106).

A une solution de 18,4 g (65,4 $10^{-3}$ moles) du composé 230105 dans 180 ml d'éthanol en présence de Pd/C à 10 % humidifié à 50 %, on fait arriver un courant d'hydrogène sous pression normale pendant 3 h 30. Après filtration et concentration, le produit est purifié par chromatographie flash (silice, éluant : acétate d'éthyle : 30 ; heptane : 70) ; RMN[1]H ($CDCl_3$) δppm : 1,05 (6H) ; 1,3 (3H) ; 3,3 (2H éch.) ; 3,7 (2H) ; 3,9 (4H) ; 6,7 (4H) ; IR (microcellule) $\nu cm^{1}$ : 3460, 3450.

Stade 4:

N-[[(Méthyl-2 dioxolane-1,3 yl-2)-2 diméthyl-1,1 éthoxy]-4 phényl] dioxa-1,4 spiro [4,5] décane

méthanamine-2 (R) (numéro de code MD230107).

8,8 g (0,035 mole) de MD 230106 ; 12,6 g (0,036 mole)de tosylate de dioxa-1,4 spiro[4,5] décane méthanol-2 (S) et 5,4 g (0,054 mole) de triéthylamine sont chauffés en bombe à 130-140 °C pendant 2 heures. Après refroidissement, le milieu réactionnel est repris dans l'acétate d'éthyle. La phase organique est lavée à l'eau saturée de NaCl et concentrée. Le produit est obtenu après chromatographie (silice, éluant : heptane : 80 ; acétate d'éthyle : 20) ; rendement 63 % ; RMN$^1$H ($CDCl_3$) $\delta$ppm : 1,1 (6H) ; 1,35 (3H) ; 1,6 (10H) ; 3,2 (2H) ; 3,6-4,5 (10H dont 1 éch.) ; 6,65 (4H) ; IR (microcellule) $\nu cm^{-1}$ : 3400 ; $[\alpha]_D^{20}$ : - 1,2 ° (C = 1, MeOH).

Stade 5 :

[(Oxo-3 diméthyl-2,2 butoxy)-4 phényl]-amino-3 propane diol-1,2 (R) (numéro de code MD230108).

A une solution de 0,7 g (1,7 $10^{-3}$ mole) du composé MD230107 dans 3,5 ml de THF, on ajoute goutte à goutte 3,5 ml d'acide chlorhydrique 6N. Après 1 h, le milieu réactionnel est versé sur l'eau et extrait à l'acétate d'éthyle. La phase organique est lavée à l'eau, séchée sur $Na_2SO_4$ et concentrée. Rendement de 80 % ; IR (microcellule) $\nu cm^{-1}$ : 3400, 1710.

Stade 6 :

[(Oxo-3 diméthyl-2,2 butoxy)-4 phényl]-3 hydroxyméthyl-5 (R) oxazolidinone-2 (numéro de code MD230109).

On chauffe au reflux une solution de 3,9 g (0,0139 mole) de MD230108 dans 50 ml de toluène et,à 90 °C, on ajoute 1,88 g (0,0159 mole) de carbonate d'éthyle, puis petit à petit 0,32 ml d'une solution de méthylate de sodium à 4,3 moles/l. Après avoir distillé l'alcool, le milieu réactionnel est concentré. Le résidu est repris dans l'acétate d'éthyle. La phase organique est lavée à l'eau, séchée et concentrée. Le produit est obtenu après chromatographie sur colonne (silice, éluant : $CH_2Cl_2$) ; F : 109 °C ; $[\alpha]_D^{20}$ : - 44,4 ° (C = 1, MeOH) ; RMN$^1$H ($CDCl_3$) $\delta$ppm : 1,25 (6H) ; 2,2 (3H) ; 3,9 (6H) ; 4,7 (1H) ; 6,9 (2H) ; 7,4 (2H) ; IR (KBr) $\nu cm^{-1}$: 3450, 1745-1725.

EXEMPLE 9 :

[(Oxo-3 diméthyl-2,2 butoxy)-4 phényl]-3 méthoxyméthyl-5 (R) oxazolidinone-2 (numéro de code MD230073).

A une suspension de 2,9 g (0,009 mole) de MD230109 dans 40 ml de toluène, on ajoute 3,8 g de soude à 50 %, 0,3 g de bromure de tétrabutylammonium et 3,6 g (0,0293 mole) de sulfate de méthyle. Après 10 mn d'agitation, le milieu réactionnel est versé sur l'eau. La phase organique est lavée à l'eau, séchée sur $Na_2SO_4$ et concentrée et le produit est obtenu après chromatographie (silice, éluant : acétate d'éthyle : 50 ; heptane : 50). F : 75 °C ; $[\alpha]_D^{20}$ : - 52 ° (C = 1, MeOH) ; RMN$^1$H ($CDCl_3$) $\delta$ppm : 1,25 (6H) ; 2,2 (3H) ; 3,6 (2H) ; 3,7-4,2 (2H) ; 3,9 (2H) ; 4,65 (1H) ; 6,8 (2H) ; 7,4 (2H) ; RMN$^{13}$C ($CDCl_3$) $\delta$ppm : Cq : 211,9 ; 155,8 ; 154,9 ; 132 ; 48,4 ; CH : 120,3 ; 115,1 ; 71,3 ; $CH_2$ : 74,9 ; 72,8 ; 47,8 ; $CH_3$ : 25,8 ; 22 ; IR (KBr) $\nu cm^{-1}$ : 1735, 1715.

EXEMPLE 10 :

[(Oxo-4 pentyl)-4 phényl]-3 hydroxyméthyl-5 (R) oxazolidinone-2 (numéro de code MD230116).

Stade 1 :

(p-nitrocinnamyl)-2 méthyl-2 dioxolane-1,3 (numéro de code MD230111).

A 62,8 mmoles de LDA dans 226,4 ml de THF, on ajoute à 0 °C une solution de 28,8 g (62,2 mmoles) de bromure de (méthyl-2 dioxolane-yl-2 éthyl-2) triphénylphosphonium dans 60 ml de DMSO, goutte à goutte. Après 1 h à 0 °C, on ajoute 7,8 g (51,6 mmoles) de p-nitro benzaldéhyde en solution dans 40 ml de THF. Le milieu réactionnel est hydrolysé par une solution saturée de $NH_4Cl$ et est extrait à l'éther éthylique. La phase organique est séchée sur $Na_2SO_4$ et concentrée. Après purification par chromatographie flash (silice, éluant : heptane : 70 ; acétate d'éthyle : 30), le produit est obtenu avec un rendement de 48 % ; RMN$^1$H ($CDCl_3$) δppm : 1,3 (3H) ; 2,6 (2H) ; 4 (4H) ; 5,7-6,5 (2H) ; 7,4 (2H) ; 8,1 (2H).

Stade 2 :

(Amino-4 cinnamyl)-2 méthyl-2 dioxolane-1,3 (numéro de code MD230112).

Une solution de 8 g (32 mmoles) du composé MD230111 dans 100 ml d'éthanol en présence de 0,8 g de Pd/C à 10 % dans un autoclave, est hydrogénée sous 5 atmosphères pendant 4 h. Après filtration, concentration, purification par chromatographie flash (silice, éluant : heptane : 50 ; acétate d'éthyle : 50), le produit est obtenu avec un rendement de 89 % ; F : < 50 °C ; RMN$^1$H ($CDCl_3$) δppm : 1,35 (3H) ; 2,4-2,8 (2H) ; 3,6 (2H éch.) ; 4 (4H) ; 5,5-6,3 (2H) ; 6,6 (2H) ; 7,2 (2H) ; IR (microcellule) $\nu cm^{-1}$ : 3460, 3440.

Stade 3 :

(Amino-4 phényl propyl)-2 méthyl-2 dioxolane-1,3 (numéro de code MD230113).

Une solution de 15,4 g (70,23 mmoles) du composé MD230112 dans 100 ml d'éthanol en présence de 1,5 g de Pd/C à 10 % dans un autoclave, est hydrogénée sous 9 atmosphères pendant 1 h. Après filtration et concentration, on obtient 15,5 g de produit attendu (liquide). RMN$^1$H ($CDCl_3$) δppm : 1,25 (3H) ; 1,6 (4H) ; 2,45 (2H) ; 3,5 (2H éch.) ; 3,85 (4H) ; 6,55 (2H) ; 6,9 (2H) ; IR (microcellule) $\nu cm^{-1}$ : 3460, 3350.

Stade 4 :

[(Méthyl-2 dioxolane-1,3 yl-2)-3 propyl]-4 phényl] dioxa-1,4 spiro [4,5] décane méthanamine-2 (R) (numéro de code MD230114).

A un mélange de 1 g (4,5 mmoles) du composé MD 230113 et 1,62 g (4,97 mmoles) de tosylate du dioxa-1,4 spiro[4,5] décane méthanol-2 (S), on ajoute 0,73 g (1 ml ; 7,23 mmoles) de triéthylamine et on chauffe à 140 °C pendant 5 h. Le milieu réactionnel est repris dans l'eau et extrait à l'acétate d'éthyle. La phase organique est lavée à l'eau salée puis séchée sur $Na_2SO_4$. On obtient le produit sous forme liquide avec un rendement de 59 % après chromatographie flash (silice, éluant : heptane : 40 ; acétate d'éthyle : 60) ; RMN$^1$H ($CDCl_3$) δppm : 1,2 (3H) ; 1,5 (10H) ; 1,6 (4H) ; 2,4 (3H) ; 3,15 (3H) ; 3,8 (4H) ; 3,6-4,5 (3H) ; IR (microcellule) $\nu cm^{-1}$ : 3400 ; $[\alpha]_D^{20}$ : - 2,9 ° (C = 1, MeOH).

Stade 5 :

[(Oxo-4 pentyl)-4 phényl]-amino propanediol-1,2 (R) (numéro de code MD230115) a été obtenu selon un mode opératoire identique à celui du stade 5 de l'exemple 8 : RMN$^1$H ($CDCl_3$) δppm : 1,8 (2H) ; 2 (3H) ; 2,4 (4H) ; 2,7-3,3 (3H) ; 3,1 (3H) ; 3,6 (2H) ; 3,3 (1H) ; 6,5 (2H) ; 6,9 (2H).

Stade 6 :

[(Oxo-4 pentyl)-4 phényl]-3 hydroxyméthyl-5 (R) oxazolidinone-2 (numéro de code MD230116) : obtenueselon un mode opératoire identique à celui du stade 6 de l'exemple 8 : F : 110 °C ; $[\alpha]_D^{20}$ : - 50,7 ° (C = 1, MeOH) ; RMN[1]H ($CDCl_3$) δppm : 1,8 (2H) ; 2,05 (3H) ; 2,2-2,7 (4H) ; 2,75 (1H) ; 3,65-4,10 (2H) ; 4,65 (1H) ; 7,1 (2H) ; 7,4 (2H ); IR (KBr) $\nu cm^{-1}$ : 3460, 1720.

EXEMPLE 11:

[(Oxo-4 pentyl)-4 phényl]-3 méthoxyméthyl-5 (R) oxazolidinone-2 (numéro de code MD230083) : a été obtenueavec un rendement de 100 % selon le mode opératoire de l'exemple 9, à partir du composé obtenu au stade 6 de l'exemple 10 : F : < 50 °C ; $[\alpha]_D^{20}$ : - 56,9 ° (C = 1, MeOH) ; RMN[1]H ($CDCl_3$) δpmm : 1,9 (2H) ; 2,1 (3H) ; 2,45 (4H) ; 3,4 (3H) ; 3,6 (2H) ; 3,9 (2H) ; 4,7 (1H) ; 7,1 (2H) ; 7,4 (2H) ; IR (KBr) $\nu cm^{-1}$ : 1750, 1710.

EXEMPLE 12 :

(Hydroxy-4 (R) pentyl)-4 phényl]-3 méthoxy méthyl-5 (R) oxazolidinone-2 (numéro de code MD230238).

Stade 1 :

Terbutyl diméthyl silyloxy méthyl-4 nitro-1 benzène (numéro de code MD230245).

A une solution de 465,4 g (3,039 moles) de paranitrobenzylalcool dans 2,5 l de DMF, on ajoute 310 g (4,559 moles) d'imidazole puis 504 g (3,347 moles) de terbutyl diméthylchlorosilane. Après 1 h d'agitation à température ambiante, le milieu réactionnel est versé sur l'eau. La phase aqueuse est extraite par le chlorure de méthylène. La phase organique est séchée sur $Na_2SO_4$ et concentrée. Huile ; RMN[1]H ($CDCl_3$) δppm : 0,2 (6H) ; 1 (9H) ; 4,9 (2H) ; 7,6 (2H) ; 8,2 (2H) ; IR (microcellule) $\nu cm^{-1}$ : 1520, 1340, 1030, 840.

Stade 2 :

Terbutyl diméthylsilyloxy méthyl-4 aniline (numéro de code MD230246).

A 772 ml de chlorure d'ammonium 0,1 N, on ajoute 77,2 g (0,288 mole) du composé précédent MD230245 et 120,9 g de poudre de fer et on chauffe à reflux 2 h. Après refroidissement, on ajoute 20 ml d'ammoniaque concentrée, le milieu réactionnel est filtré et extrait au toluène. La phase organique est lavée à l'eau, séchée sur $Na_2SO_4$ et concentrée ; Eb 0,01 mm Hg:88-93 °C ; RMN[1]H ($CDCl_3$) δpm : 0,2 (6H) ; 1,05 (9H) ; 3,6 (2H) ; 4,8 (2H) ; 6,75 (2H) ; 7,2 (2H) ; IR (microcellule) $\nu cm^{-1}$ : 3450, 3350.

Stade 3 :

[(Terbutyl diméthyl silyoxy méthyl)-4 phényl]-3 méthoxyméthyl-5 (R) oxazolidinone-2 (numéro de code MD 230247)

A une solution de 43,8 g (0,168 mole) de tosylate de méthoxy-3 propanediol-1,2 (R) dans 200 ml de toluène, on ajoute 130 ml d'une solution toluénique de phosgène 1,93 molaire puis, goutte à goutte, 37,8 g (0,252 mole) de diéthylaniline.Après refroidissement, on ajoute de l'eau glacée et la phase organique est décantée et séchée sur $Na_2SO_4$. Cette solution est alors additionnée à une solution de 40 g (0,168 mole) de MD230246 (stade 2) et de 20,5 g (0,168 mole) de diméthylamino-4 pyridine dans 600 ml de toluène.

Après 1/2 h d'agitation, le milieu réactionnel est versé sur l'eau et la phase organique est lavée avec une solution de bicarbonate de sodium puis une solution saturée en NaCl. Après concentration, le produit obtenu (84,5 g) est mis en solution dans 800 ml d'éthanol auquel est ajouté 12,2 g (0,218 mole) de KOH en pastilles. Après 1/2 h d'agitation, le milieu réactionnel est versé sur l'eau et extrait au chlorure de méthylène. La phase organique est séchée sur $Na_2SO_4$ et concentrée. Le produit est obtenu après chromatographie (silice, éluant : acétate d'éthyle : 30 ; heptane : 70) avec un rendement de 63 % ; $[\alpha]_D^{20}$ : - 46,2 ° (C = 1, $CH_3OH$) ; IR (KBr) $\nu cm^{-1}$ : 1755, 1735; RMN$^1$H ($CDCl_3$) δppm : 0 (6H) ; 1 (9H) ; 3,4 (3H) ; 3,6 (2H) ; 3,8-4,2 (2H) ; 4,7 (3H) ; 7,5 (4H) ; F < 50 ° C.

Stade 4:

[(Hydroxyméthyl-4) phényl-]-3 méthoxyméthyl-5 (R) oxazolidinone-2 (numéro de code MD230248).

Une solution de 29,2 g (0,083 mole) de MD230247 et 7,8 g (0,025 mole) de fluorure de tétrabutylammonium trihydraté dans 200 ml de THF est agitée 12 h à température ordinaire et concentrée. Le produit obtenu après chromatographie (silice, éluant : acétate d'éthyle : 50, heptane : 50) ; F : 65 ° C ; IR (KBr) $\nu cm^{-1}$ : 3400, 1750, 1720. ; RMN$^1$H ($CDCl_3$) δppm : 2,4 (1H éch.) ; 3,35 (3H) ; 3,6 (2H) ; 3,8-4,2 (2H) ; 4,6 (2H) ; 7,35 (4H).

Stade 5 :

(carboxyaldéhyde-4 phényl)-3 méthoxyméthyl-5 (R) oxazolidinone-2 (numéro de code MD230256).

A une solution refroidie à - 60 ° C de 12,46 g (0,0982 mole) de chlorure d'oxalyle dans 80 ml de chlorure de méthylène, on introduit en 20 mn une solution de 12,76 g (0,1630 mole) de DMSO dans 80 ml de chlorure de méthylène. 40 mn plus tard, on ajoute une solution de 19,6 g (0,0818 mole) de MD230248 dans 80 ml de chlorure de méthylène puis 1,4 g (0,409 mole) de triéthylamine. Après retour à température ambiante, on ajoute 300 ml d'eau. La phase organique est lavée à l'eau, séchée et concentrée. Le produit a été obtenu après purification par chromatographie (silice, éluant : acétate d'éthyle : 70, heptane : 30) avec un rendement de 80 % ; F : 96 ° C ; $[\alpha]_D^{20}$ : - 73,4 ° (C = 1, $CH_2Cl_2$) ; IR (KBr) $\nu cm^{-1}$ : 1740, 1690 ; RMN$^1$H ($CDCl_3$) δppm : 3,4 (3H) ; 3,7 (2H) ; 3,8-4,3 (2H) ; 4,8 (1H) ; 7,8 (4H) ; 9,8 (1H).

Stade 6 :

[(Hydroxy-4 (R) pentyl)-4 phényl]-3 méthoxyméthyl-5 (R) oxazolidinone-2 (numéro de code MD230238).

Une solution de 3,3 g (0,00712 mole) d'iodure d'hydroxy-2 (R) propyl triphénylphosphonium (Helv. Chim. Acta, 59, 755-757, 1976),de 1,34 g (0,00569 mole) de MD230256 et 2,9 g (0,0213 mole) de $K_2CO_3$ dans 10 ml de dioxanne et 1,5 ml de formamide est chauffée a reflux 20 h. Après filtration et concentration, le produit insaturé obtenu est purifié en le dissolvant dans 30 ml de DMF et on ajoute 0,58 g d'imidazole et 0,94 g (0,00625 mole) de terbutyl diméthylchlorosilane. Après 24 h d'agitation, le milieu réactionnel est versé sur l'eau. Le produit silylé est extrait au chlorure de méthylène et purifié par chromatographie (silice, éluant : acétate d'éthyle : 50 ; heptane : 50) avec un rendement de 36 %. 0,84 g du produit résultant est mis en solution dans 15 ml de THF en présence de 0,65 g de fluorure de tétrabutylammonium pendant 12 h. Après concentration et purification par chromatographie (silice, éluant : acétate d'éthyle : 70 ; heptane : 30), 0,53 g (0,0018 mole) de produit insaturé purifié en solution dans 10 ml de méthanol en présence de palladium sur carbone à 10 % (50 % humide) est hydrogéné sous pression normale. Le produit attendu est obtenu avec un rendement de 55 % après chromatographie (silice, acétate d'éthyle : 60 ; heptane : 40) ; $[\alpha]_D^{20}$ : - 45,8 ° (C = 1, $CH_2Cl_2$) ; IR (KBr) $\nu cm^{-1}$ : 3400, 1735 ; F : 47 ° C ; RMN$^1$H ($CDCl_3$) δppm : 1,2 (3H) ; 1,5 (4H) ; 1,8 (1H éch.) ; 2,6 (2H) ; 3,4 (3H) ; 3,6 (2H) ; 3,7-4,2 (3H) ; 4,7 (1H) ; 7,2 (2H) ; 7,4 (2H).

De la même façon a été obtenu : [(hydroxy-4 (S) pentyl)-4 phényl]-3 méthoxyméthyl-5 (R) oxazolidinone-2 (numéro de code MD230239) ; F : 53 ° C ; $[\alpha]_D^{20}$ : - 35,9 ° (C = 1, $CH_2Cl_2$) ; IR (KBr)

$\nu cm^{-1}$ : 3400, 1740 ; RMN¹H ($CDCl_3$) δppm : 1,1 (3H) ; 1,6 (5H dont 1 éch.) ; 3,4 (3H) ; 3,6 (2H) ; 3,7-4,2 (3H) ; 4,7 (1H) ; 7,1 (2H) ; 7,4 (2H).

**Exemple 13 :**

[[(phénylméthyl-2 dioxolane-1,3 yl-2)-3 propyl]-4 phényl]-3 méthoxyméthyl-5 (R) oxazolidinone-2 (numéro de code MD 360334)

Stade 1

[(phénylméthyl)-2 dioxolane-1,3 yl-2]-2 éthanol (numéro de code MD 360370)

Obtenu selon la méthode décrite au stade 1 de l'exemple 8, à partir de l'ester éthylique de l'acide [-(phénylméthyl)-2 dioxolane-1,3 yl-2] acétique (Synthesis 451, 1982) : IR (microcellule) $\nu$ $cm^{-1}$ : 3440-3400 ; RMN¹H ($CDCl_3$) δ ppm : 1,9 (2H) ; 2,8 (3H dont 1 éch.) ; 3,5 - 4 (6H) ; 7,2 (5H).

Stade 2

(phénylméthyl)-2 (bromo-2 éthyl)-2 dioxolane-1,3 (numéro de code MD 360371)

A une solution de 37,8 g (0,181 mole) de MD 360370 dans 200 ml de $CH_2Cl_2$, on ajoute 120,4 g (0,363 mole) de $CBr_4$, puis petit à petit 95,2 g (0,363 mole) de triphénylphosphine, puis le milieu réactionnel est agité à température ambiante pendant 1/2 heure . Après filtration, la phase organique est concentrée. Rendement : 81 % ; IR (microcellule)$\nu$ $cm^{-1}$: 3020, 2960, 2880, 1605 ; RMN¹H ($CDCl_3$) δ ppm : 2,2 (2H) ; 2,8 (2H) ; 3,4 (2H) ; 3,8 (4H) ; 7,2 (5H).

Stade 3

Bromure de [[(phénylméthyl)-2 dioxolane-1,3 yl-2]-2 éthyl] triphénylphosphonium (numéro de code MD 360372)

A une solution de 33 g (0,1217 mole) de MD 360371 dans 200 ml de dioxane, on ajoute 31 g (0,1217 mole) de triphénylphosphine et chauffe le mélange 20 heures. Après refroidissement, le précipité est filtré et lavé au dioxane et à l'éther éthylique. Rendement : 81 % ; F = 225° C ; RMN¹H ($CDCl_3$) δ ppm : 1,6-2,2 (2H) ; 3 (2H) ; 3,2-4,2 (6H) ; 7,2 (5H) ; 7,5-7,9 (15H).

De la même manière ont été obtenus :

- le bromure de [(phényl-2 dioxolane-1,3-yl-2)-2 éthyl] triphénylphosphonium [F : 228° C ; RMN¹H ($CDCl_3$) δppm : 2-2,5 (2H) ; 3,4-4,4 (6H) ; 7,4 (5H) ; 7,6-8 (15 H)] à partir du phényl-2 (bromo-2 éthyl)-2 dioxolane-1,3 (Tétrahedron Letters, 1987, 28, 1397).
- le bromure de [cyclohexyl-2 dioxolane-1,3-yl-2)-2 éthyl] triphénylphosphonium, à partir du cyclohexyl-2 (bromo-2 éthyl)-2 dioxolane-1,3 : Liq, IR (microcellule)$\nu$ $cm^{-1}$: 2920-2850, 1440-1110 ; RMN¹H ($CDCl_3$) δppm : 0,9-2,3 (13H) ; 3-4,2 (6H) ; 7,6-8 (15H).

Stade 4

(Para-nitrocinnamyl)-2 (phénylméthyl)-2 dioxolane-1,3 (numéro de code MD 360373)

Obtenu selon le mode opératoire du stade 1 de l'exemple 10 : liquide ; IR (microcellule)$\nu$ $cm^{-1}$ : 1595,

1510, 1340 ; RMN$^1$H ($CDCl_3$) δppm : 2,6 (2H) ; 3 (2H) ; 3,9 (4H) ; 5,8-6,8 (2H) ; 7,3 (5H) ; 7,4 (2H) ; 8,2 (2H).

De la même façon ont été obtenus :

- (para-nitrocinnamyl)-2 phényl-2 dioxolane-1,3
(numéro de code MD 360384)
IR (microcellule)ν $cm^{-1}$ : 1595, 1510, 1340 ; RMN$^1$H ($CDCl_3$) δppm : 2,8-3 (2H) ; 3,6-4,2 (4H) ; 5,6-6,8 (2H) ; 7,15-7,65 (7H) ; 8,1 (2H) ; F = 82° C ;
- (para-nitrocinnamyl)-2 cyclohexyl-2 dioxolane-1,3
(numéro de code MD 360416)
IR (microcellule)ν $cm^{-1}$ : 2920-2850, 1595-1510, 1390 ; RMN$^1$H ($CDCl_3$) δppm : 0,8-2,1 (11H) ; 2,5-2,8 (2H) ; 4 (4H) ; 5,7-6,7 (2H) ; 7,45 (3H) ; 8,2 (2H).

Stade 5

-[(amino-4 phényl)-3 propyl]-2 (phénylméthyl)-2 dioxolane-1,3 (numéro de code MD 360374)

Obtenu par hydrogénation de MD 360373 selon le mode opératoire du stade 3 de l'exemple 10.

F : 55° C ; IR (KBr) ν$cm^{-1}$ : 3450-3360, 1620-1510 ; RMN$^1$H ($CDCl_3$) δ ppm : 1,65 (4H) ; 2,45 (2H) ; 2,85 (2H) ; 3,45 (2H éch.) ; 3,45-4 (4H) ; 6,5 (2H) ; 6,9 (2H) ; 7,2 (5H).

De la même manière ont été obtenus :

- [(amino-4 phényl)-3 propyl]-2 phényl-2 dioxolane-1,3
(numéro de code MD 360385)
F = 68° C ; IR (KBr)ν $cm^{-1}$ : 3440-3360, 1630-1610, 1515; RMN$^1$H ($CDCl_3$) δ ppm : 1,4-2,2 (4H) ; 2,4 (2H) ; 3,45 (2H) ; 3,6-4,15 (4H) ; 6,5 (2H) ; 6,9 (2H) ; 7,2-7,6 (5H) ;
- [(amino-4 phényl)-3 propyl]-2 cyclohexyl-2 dioxolane-1,3
(numéro de code MD 360417)
IR (microcellule)ν$cm^{-1}$ : 3440-3360, 1625 ; RMN$^1$H ($CDCl_3$) δppm : 0,8-2 (11H) ; 2,4 (2H) ; 3,45 (2H éch.) ; 3,8 (4H) ; 6,5 (2H) ; 6,9 (2H).

Stade 6

[[(éthoxycarbonylamino)-4 phényl]-3 propyl]-2 (phénylméthyl)-2 dioxolane-1,3 (numéro de code MD 360375)

Obtenu par action du chloroformiate d'éthyle ($55.10^{-3}$ mole) sur une solution de MD 360374 ($10^{-3}$ mole) en solution dans un mélange THF/eau (90/10) en présence de bicarbonate de sodium (6,3g) RMN$^1$H ($CDCl_3$) δppm : 1,25 (3H) ; 1,65 (4H) ; 2,5 (2H) ; 2,85 (2H) ; 3,7 (4H) ; 4,2 (2H) ; 6,9 (1H éch.) ; 7-7,4 (9H).

De la même manière ont été obtenus :

- [[(éthoxycarbonylamino)-4 phényl]-3 propyl]-2 phényl-2 dioxolane-1,3
(numéro de code MD 360386)
RMN$^1$H ($CDCl_3$) δ ppm : 1,25 (3H) ; 1,4-2,2 (4H) ; 2,5 (2H) ; 3,5-4 (4H) ; 4,2 (2H) ; 6,6-7,6 (10H dont 1 éch.) ; F = 66° C.
- [[(éthoxycarbonylamino)-4 phényl]-3 propyl]-2 cyclohexyl-2 dioxolane-1,3
(numéro de code MD 360420)
F = 70° C ; IR (KBr)ν $cm^{-1}$ : 3360, 1705

Stade 7

[[(phénylméthyl-2 dioxolane-1,3 yl-2)-3 propyl]-4 phényl]-3 méthoxyméthyl-5 (R) oxazolidinone-2 (numéro de code MD 360334)

Obtenue par réaction à 160° C sous agitation de MD 360375 (3,6. $10^{-3}$ mole), $K_2CO_3$ ($0,72.10^{-3}$mole)

et MD 360287 (exemple 15) pendant 3 heures.
RMN[1]H ($CDCl_3$) δ ppm : 1,6 (4H) ; 2,5 (2H) ; 2,85 (2H) ; 3,4 (3H) ; 3,45-4,2 (8H) ; 4,65 (1H) ; 6,9-7,6 (9H) ; $[\alpha]_D^{20}$ : -33,2° (C = 1, $CH_2Cl_2$)

De la même manière ont été obtenus :
-[[(phényl-2 dioxolane-1,3 yl-2)-3 propyl]-4 phényl]-3 méthoxyméthyl-5 (R) oxazolidinone-2 (numéro de code MD 360332)
RMN[1]H ($CDCl_3$) δ ppm : 1,4-2,1 (4H) ; 2,55 (2H) ; 3,4 (3H) ; 3,6 (2H) ; 3,6-4,2 (6H) ; 4,65 (1H) ; 6,95-7,95 (IR (KBr) $\nu cm^{-1}$ : 1750 ; $[\alpha]_D^{20}$ = - 31,9° (C = 1, $CH_2Cl_2$)
- [[(cyclohexyl-2 dioxolane-1,3 yl-2)-3 propyl]-4 phényl]-3 méthoxyméthyl-5 (R) oxazolidinone-2 (numéro de code 360354) ; F : 86° C.

**Exemple 14 :**

[(phényl-5 oxo-4 pentyl)-4 phényl]-3 méthoxyméthyl-5 (R) oxazolidinone-2 (MD 360394)

Obtenue à partir de MD 360334 (exemple 13) soumis au mode opératoire de la méthode 2 de l'exemple 4 . Rendement : 58 % ; F : 105° C ; $[\alpha]_D^{20}$ : - 35,9° (C = 1, $CH_2Cl_2$) ; RMH[1]H ($CDCl_3$) δ ppm : 1,9 (2H) ; 2,5 (4H) ; 3,4 (3H) ; 3,6 (2H) ; 3,65 (2H) ; 3,95 (2H) ; 4,7 (1H) ; 7,1 (2H) ; 7,25 (5H) ; 7,45 (2H) ; IR (KBr) $\nu cm^{-1}$ : 1740, 1710.

De la même manière on obtient, à partir des dioxolanes correspondants :
- [(phényl-4 oxo-4 butyl)-4 phényl]-3 méthoxyméthyl-5 (R) oxazolidinone-2 (MD 360401) ;
IR (microcellule)$\nu cm^{-1}$ : 1750-1735 ; RMN[1]H ($CDCl_3$) δppm : 2,05 (2H) ; 2,5-3,1 (4H) ; 3,4 (3H) ; 3,6 (2H) ; 3,8-4,2 (2H) ; 4,65 (1H) ; 7-7,6 (7H) ; 7,9 (2H) ; F = 83° C.
- [(cyclohexyl-4 oxo-4 butyl)-4 phényl]-3 méthoxyméthyl-5 (R) oxazolidinone-2 (numéro de code 360399) F = 80° C ; RMN[1]H ($CDCl_3$) δ ppm : 0,9-2,2 (13H) ; 2,2-2,7 (4H) ; 3,4 (3H) ; 3,6 (2H) ; 3,7-4,2 (2H) ; 4,6 (1H) ; 7,1 (2H) ; 7,4 (2H).

**Exemple 15 :**

Méthoxyméthyl-4 dioxolane-1,3 one-2 (S) (numéro de code MD 360287)

On chauffe un mélange de 14 g (0,132 mole) de méthoxy-3 propanediol-1,2 (R) et de 31,16 g (0,264 mole) de carbonate de diéthyle en présence de 0,108 g de NaH à 50 %, jusqu'à distillation de l'alcool formé. Lorsque la réaction est complète, le produit attendu est distillé $Eb_{0,3}$ : 117° C ; Rendement : 93 % ; $[\alpha]_D^{20}$ : - 32,2° (C = 1, $CH_2Cl_2$) ; IR (microcellule) $\nu cm^{-1}$ : 1790.

**Exemple 16 :**

cyclohexyl-2 (bromo-2 éthyl)-2 dioxolane-1,3 (numéro de code 360414)

Stade 1

bromo-3 cyclohexyl-1 propanone

Dans une solution de cyclohexyl-1 one-1 propène-2 (0,255 mole) dans 200 ml de $CH_2Cl_2$, refroidie à 10-15° C, on fait barboter HBr gazeux. Quand la réaction est achevée, on lave le milieu réactionnel avec une solution aqueuse saturée de $NaHCO_3$, sèche sur $Na_2SO_4$ et concentre pour obtenir le produit attendu sous la forme d'une huile.

Stade 2

cyclohexyl-2 (bromo-2 éthyl)-2 dioxolane-1,3

On porte au reflux, avec élimination de l'eau formée, une solution du composé obtenu au stade précédent (0,223 mole) dans 600 ml de benzène, cette solution comprenant en outre 0,58 mole d'éthylène glycol et 2,5 g d'acide para-toluène sulfonique. Après 3 heures 30 mn de réaction, on verse dans une solution saturée de NaCl, sèche la phase organique sur $Na_2SO_4$, concentre et purifie par chromatographie (silice, éluant : heptane 60 - $CH_2Cl_2$40).

**Exemple 17 :**

Stade 1 :

Diméthyl-2,2 méthoxyméthyl-4 (S) dioxolane (numéro de code MD370486).

A 910 ml d'eau, on ajoute 910 g de NaOH en pastilles puis à température ambiante 5 l de chlorure de méthylène ,44,4 g (0,195 mole) de chlorure de benzyltriéthylammonium, 8558,6 g (6,5 moles) de diméthyl-2,2 hydroxyméthyl-3 (S) dioxolane et 1229,5 g (9,75 moles) de sulfate de diméthyle. Le milieu réactionnel est agité 12 h et versé sur l'eau. La phase organique est concentrée. Le produit est distillé : Eb : 45 °C sous 10 mm Hg ; $[\alpha]_D^{20}$ : + 7,9° (C = 4, $CH_3OH$) ; IR (microcellule) $\nu cm^{-1}$ : 2995, 2940, 2820, 1380, 1370, 840 ; RMN$^1$H ($CDCl_3$) δppm : 1,2 (3H) ; 1,4 (3H) ; 3,35 (3H) ; 3,4-4,4 (3H) ; 4 (2H).

Stade 2 :

Méthoxy-3 propane diol-1,2 (R) (numéro de code MD370487).

On chauffe une solution de 950,3 g (6,5 moles) de MD370486 dans 4510 ml d'eau à 60 °C et on ajoute 3,2 ml d'acide chlorhydrique concentré. Puis on ajoute 9 ml de triéthylamine et le milieu réactionnel est concentré et distillé avec un rendement de 84 % ; Eb : 66 °C sous 1 mm Hg ; $[\alpha]_D^{20}$ : - 6,4° (C = 4, $CH_3OH$) ; IR (microcellule) $\nu cm^{-1}$ : 3300,3500, 2960, 2945, 2910 ; RMN$^1$H (DMSO $d_6$) δppm : 3,2-3,7 (8H) ; 4,5 (2H éch.).

**Exemple 18 :**

[(oxo-3 pentènyl-1)-4 phényl]-3 méthoxyméthyl-5 (R) oxazolidinone-2 (numéro de code MD 360393)

Stade 1 :

[(méthyl-2 dioxolane-1,3 yl-2 propènylène-1)-4 phényl]-3 méthoxyméthyl-5 (R) oxazolidinone-2 (numéro de code 360392)

On chauffe un mélange de 0,470 g ($2.10^{-3}$ mole) de MD 230256 (ex. 12), 0,414 g ($3.10^{-3}$ mole) de $K_2CO_3$, 1,14 g ($2,5.10^{-3}$ mole) du composé phosphonium mis en oeuvre au stade 1 de l'exemple 10 en solution dans 2 ml de dioxane et 0,072 ml d'eau à 80° C pendant 3 h. Le milieu réactionnel est versé sur l'eau et extrait à l'acétate d'éthyle. La phase organique est séchée sur $MgSO_4$ et concentrée. Le produit est obtenu après chromatographie (silice, heptane : 40, acétate d'éthyle : 60), avec un rendement de 31 %. RMN$^1$H ($CDCl_3$) δ ppm : 1,3 (3H) ; 2,6 (2H) ; 3,4 (3H) ; 3,6 (2H) ; 4 (6H) ; 4,7 (1H) ; 5,7 (1H) ; 6,4 (1H) ; 7,7-7,6 (4H).

Stade 2 : Composé de numéro de code MD 360393

Obtenu suivant le mode opératoire de la méthode 2 de l'exemple 4 : IR (microcellule)$\nu$cm$^{-1}$ : 1748, 1712, 1606 ; RMN$^1$H ($CDCl_3$) $\delta$ppm : 2,2 (3H) ; 3,4 (5H) ; 3,6 (2H) ; 3,9 (2H) ; 4,7 (1H) ; 5,7-6,8 (2H) ; 7,1-7,7 (4H).

Les dérivés de formule (I) ont été étudiés chez l'animal de laboratoire et ont montré des activités pharmacologiques notamment dans le domaine psychotrope, en particulier comme anxiolytiques et antidépresseurs potentiels.

L'activité antidépressive a été mise en évidence par le test de potentialisation du 5-HTP chez le rat selon le protocole décrit par M. JALFRE, B. BUCHER, A. COSTON, G. MOCQUET et R.D. PORSOLT : Arch. Int. Pharmacodyn. (1982), 259; 194-221. On détermine chez le rat la dose de produit qui, administrée par voie orale, provoque chez 50 % des animaux ($DE_{50}$) l'apparition de tremblements généralisés ou des stéréotypies (pianotage, mouvements de tête) consécutifs à l'administration par voie intrapéritonéale 1 h après le premier traitement d'hydroxy-5 tryptophane (5-HTP). Les résultats obtenus avec certains composés selon l'invention dans le test précédent sont rassemblés, à titre d'exemple dans le tableau ci-après qui donne par ailleurs la toxicité aiguë ($DL_{50}$) de certains des composés testés et qui est estimée chez la souris selon la méthode de J.T. LITCHFIELD et F. WILCOXON (J. Pharmacol. Exp. Ther., (1949), 96, 99.

TABLEAU

| COMPOSE TESTE NUMERO DE CODE | $DE_{50}$MG/KG | $DL_{50}$P.O.MG/KG |
|---|---|---|
| MD370268<br>MD370298<br>MD230050<br>MD230083<br>TOLOXATONE | 0,72<br>1,6<br>1,2<br>1,9<br>30 | >3 500 |

Les résultats qui précèdent montrent que les composés objet de la présente demande peuvent être utilisés pour la préparation de médicaments psychotropes et notamment d'anxiolytiques et d'antidépresseurs potentiels, ces médicaments trouvant leur emploi en thérapeutique, notamment pour le traitement des états dépressifs endogène et exogène.

Ces médicaments peuvent être administrés à l'homme ou à tout animal à sang chaud, sous diverses formes pharmaceutiques bien connues dans la technique et notamment sous la forme de compositions formulées en vue de leur administration par voie orale, injectable ou rectale.

Pour l'administration par voie orale, lesdites compositions peuvent prendre la forme de comprimés, dragées ou gélules préparées par les techniques habituelles utilisant des supports et excipients connus tels que des agents liants, des charges, des lubrifiants et des agents de désintégration ; elles peuvent également prendre la forme de solutions, sirop ou suspensions.

Pour l'administration sous forme de soluté injectable, les compositions selon l'invention peuvent prendre la forme de solutions, suspensions ou émulsions injectables comprenant un véhicule liquide huileux ou aqueux acceptable.

Pour l'administration par voie rectale, les compositions peuvent prendre la forme de suppositoire comprenant les bases habituelles pour suppositoires.

La dose thérapeutique active des principes actifs, c'est-à-dire des composés (I) et leurs sels pharmaceutiquement acceptables, dépend, notamment de la voie d'administration, du poids corporel du patient et de la puissance thérapeutique des principes actifs mis en oeuvre.

Généralement, par voie orale, les doses administrées pourront atteindre 10 mg/kg/jour de principe actif (en une ou plusieurs prises) ; par voie injectable, elles pourront atteindre 1 mg/kg/jour (en une ou plusieurs prises) ; par voie rectale, elles pourront atteindre 5 mg/kg/jour de principe actif (en un ou plusieurs suppositoires).

## Revendications

1. Dérivés répondant à la formule :

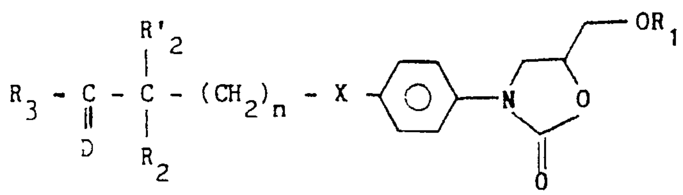

(I)

où :

- $R_1$ représente H ou alkyle en $C_1$-$C_4$ ;
- X représente un atome d'oxygène, un groupe méthylène ou un groupe -CH = CH- ;
- n prend la valeur 1 ou 2 quand X est un atome d'oxygène ou un groupe méthylène et la valeur 0 ou 1 quand X représente le groupe -CH = CH- ;
- D représente un atome d'oxygène ou un groupe NOR où R = H ou alkyle en $C_1$-$C_4$ ;
- $R_3$ représente un groupe alkyle en $C_1$-$C_4$,cycloalkyle en $C_3$-$C_7$, phényle ou benzyle ;
- $R_2$ et $R'_2$ représentent chacun indépendamment l'un de l'autre, un atome d'hydrogène ou un groupe alkyle en $C_1$-$C_4$, cycloalkyle en $C_4$-$C_7$, phényle ou benzyle ; et
- $R'_2$ et $R_3$ peuvent en outre former ensemble une chaîne $-(CH_2)_3-$ ou $-(CH_2)_4-$,

ces dérivés se présentant sous la forme de diastéréoisomères ou d'énantiomères ou sous forme cis ou trans ou encore sous la forme d'un mélange de toutes ces formes, y compris les formes racémiques, à l'exception des racémiques pour lesquels $R_1$ = $CH_3$, X = oxygène, $R_2$ = $R'_2$ = H, n = 1 ou 2 et D représente oxygène.

2. Dérivés selon la revendication 1, pour lesquels $R_1$ = H ou $CH_3$ ; X = oxygène ou $CH_2$ ; n = 1 ou 2 ; $R_2$ = $R'_2$ = H ou $CH_3$ ; $R_3$ = alkyle en $C_1$-$C_4$ ; et D représente oxygène, N-OH ou N-$OCH_3$.

3. Dérivés selon la revendication 1, pour lesquels $R_1$ = $CH_3$ ; X = oxygène ; n = 1 ou 2 ; $R_2$ = $R'_2$ = H ; $R_3$ = $CH_3$ ; et D représente oxygène.

4. Dérivés selon la revendication 1, pour lesquels $R_1$ = $CH_3$ ; X = oxygène ; n = 1 ou 2 ; $R_2$ = $R'_2$ = H ; $R_3$ = $CH_3$ ; et D représente N-OH(E).

5. Dérivés selon la revendication 1, pour lesquels $R_1$ = $CH_3$ ; X = méthylène ; n = 1 ou 2 ; $R_2$ = $R'_2$ = H ; $R_3$ = $CH_3$ ; et D représente oxygène.

6. Dérivés selon la revendication 1, pour lesquels $R_1$ = $CH_3$ ; X = méthylène ; n = 1 ou 2 ; $R_2$ = $R'_2$ = H ; $R_3$ = $CH_3$ ; et D représente N-OH(E).

7. Dérivés selon la revendication 1, pour lesquels $R_1$ = $CH_3$ ; X représente -CH = CH- ; n = 0 ou 1 ; $R_2$ = $R'_2$ = H ; $R_3$ = $CH_3$ ; et D représente oxygène.

8. Dérivés selon la revendication 1, pour lesquels $R_1$ = $CH_3$ ; X représente CH = CH ; n = 0 ou 1 ; $R_2$ = $R'_2$ = H ; $R_3$ = $CH_3$ ; et D représente N-OH(E).

9. Dérivé selon la revendication 1, pour lequel $R_1$ = $CH_3$ ; X = méthylène ; n = 1 ; $R_2$ = $R'_2$ = H ; $R_3$ = $CH_3$ ; et D représente oxygène.

10. Dérivés selon l'une des revendications 3 à 9, pour lesquels l'atome de carbone asymétrique est de configuration (R).

11. Composition pharmaceutique, caractérisée en ce qu'elle comprend un excipient physiologiquement acceptable et au moins un composé choisi parmi ceux objet de l'une quelconque des revendications 1 à 10.

12. Utilisation des composés objet des revendications 1 à 10 pour la préparation de médicaments.

13. Procédé de préparation des dérivés de formule (I) selon la revendication 1, caractérisé en ce qu'il comprend :

a) l'hydrolyse des composés de formule :

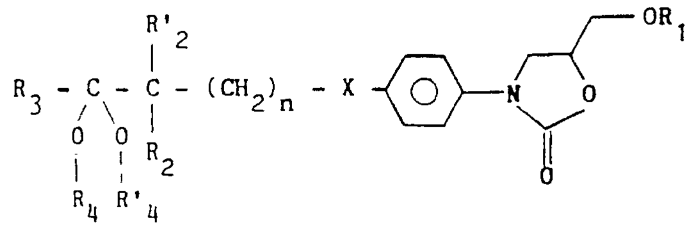

où $R_1$, X, n, $R_2$, $R'_2$ et $R_3$ ont la même signification que dans la formule (I), et $R_4$ et $R'_4$ représentent indépendamment l'un de l'autre alkyle en $C_1$-$C_4$ ou forment ensemble une chaîne $-(CH_2)_2-$ ou $-(CH_2)_3$ ;

b) l'oxydation des composés de formule :

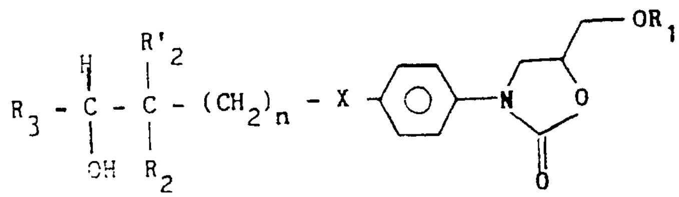

où les différents paramètres ont la même signification que dans la formule (I) ;

c) la condensation d'un carbonate d'alkyle en $C_1$-$C_4$ sur les composés de formule :

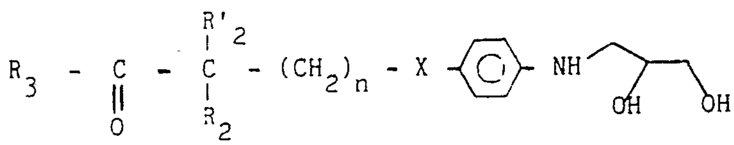

où n , X, $R_2$, $R'_2$ et $R_3$ ont les mêmes significations que dans la formule (I) ; ou

d) l'alcoylation par un halogénure d'alkyle en $C_1$-$C_4$ descomposés de formule :

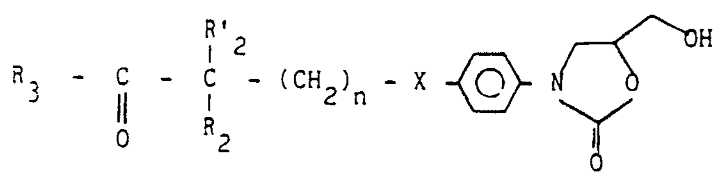

où n, X, $R_2$, $R'_2$ et $R_3$ ont les mêmes significations que dans la formule (I).

Revendications pour les Etats contractants suivants: ES, GR

1. Procédé de préparation des dérivés de formule :

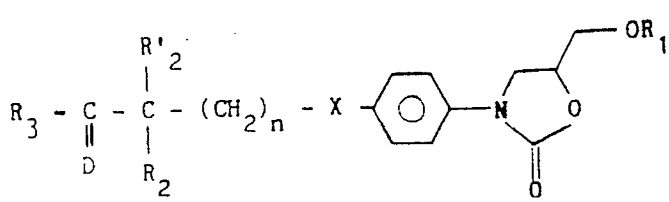

(I)

où :

- $R_1$ représente H ou alkyle en $C_1$-$C_4$ ;
- X représente un atome d'oxygène, un groupe méthylène ou un groupe -CH=CH- ;
- n prend la valeur 1 ou 2 quand X est un atome d'oxygène ou un groupe méthylène et la valeur 0 ou 1 quand X représente le groupe -CH=CH- ;
- D représente un atome d'oxygène ou un groupe NOR où R = H ou alkyle en $C_1$-$C_4$ ;
- $R_3$ représente un groupe alkyle en $C_1$-$C_4$,cycloalkyle en $C_3$-$C_7$, phényle ou benzyle ;
- $R_2$ et $R'_2$ représentent chacun indépendamment l'un de l'autre, un atome d'hydrogène ou un groupe alkyle en $C_1$-$C_4$, cycloalkyle en $C_4$-$C_7$, phényle ou benzyle ; et
- $R'_2$ et $R_3$ peuvent en outre former ensemble une chaîne $-(CH_2)_3-$ ou $-(CH_2)_4-$,

ces dérivés se présentant sous la forme de diastéréoisomères ou d'énantiomères ou sous forme cis ou trans ou encore sous la forme d'un mélange de toutes ces formes, y compris les formes racémiques, à l'exception des racémiques pour lesquels $R_1$ = $CH_3$, X = oxygène, $R_2$ = $R'_2$ = H, n = 1 ou 2 et D représente oxygène, caractérisé en ce qu'il comprend :

a) l'hydrolyse des composés de formule :

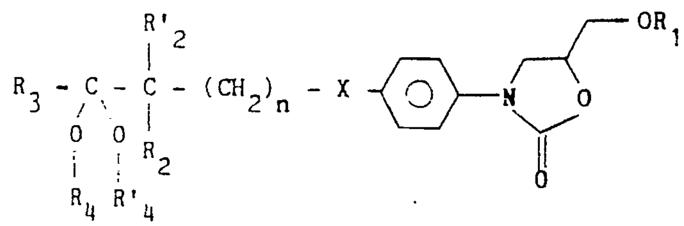

où $R_1$, X, n, $R_2$, $R'_2$ et $R_3$ ont la même signification que dans la formule (I), et $R_4$ et $R'_4$ représentent indépendamment l'un de l'autre alkyle en $C_1$-$C_4$ ou forment ensemble une chaîne $-(CH_2)_2-$ ou $-(CH_2)_3$ ;

b) l'oxydation des composés de formule :

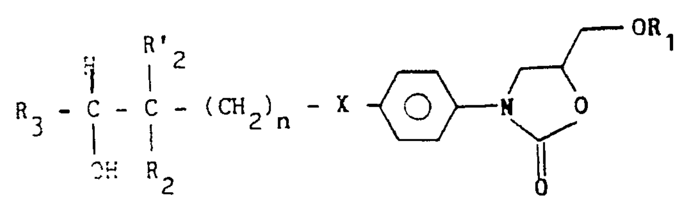

où les différents paramètres ont la même signification que dans la formule (I) :

c) la condensation d'un carbonate d'alkyle en $C_1$-$C_4$ sur les composés de formule :

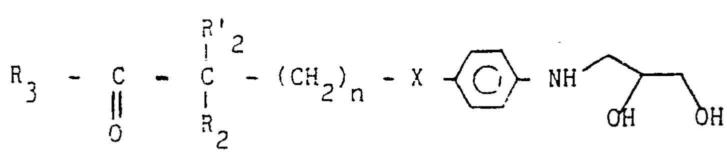

où n , X, $R_2$, $R'_2$ et $R_3$ ont les mêmes significations que dans la formule (I) ; ou

d) l'alcoylation par un halogénure d'alkyle en $C_1$-$C_4$ descomposés de formule :

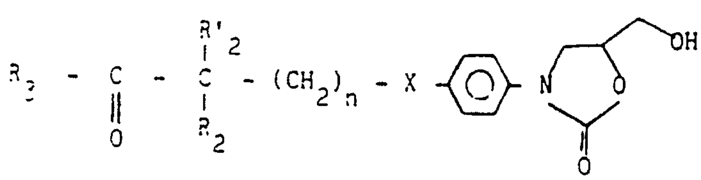

où n, X, $R_2$, $R'_2$ et $R_3$ ont les mêmes significations que dans la formule (I).

2. Procédé selon la revendication 1, pour la préparation des dérivés de formule (I) pour lesquels $R_1$ = H ou $CH_3$ ; X = oxygène ou $CH_2$ ; n = 1 ou 2 ; $R_2$ = $R'_2$ = H ou $CH_3$ ; $R_3$ = alkyle en $C_1$-$C_4$ ; et D représente oxygène, N-OH ou N-$OCH_3$.

3. Procédé selon la revendication 1, pour la préparation des dérivés de formule (I) pour lesquels $R_1$ = $CH_3$ ; X = oxygène ; n = 1 ou 2 ; $R_2$ = $R'_2$ = H ; $R_3$ = $CH_3$ ; et D représente oxygène.

4. Procédé selon la revendication 1, pour la préparation des dérivés de formule (I) pour lesquels $R_1$ = $CH_3$ ; X = oxygène ; n = 1 ou 2 ; $R_2$ = $R'_2$ = H ; $R_3$ = $CH_3$ ; et D représente N-OH(E).

5. Procédé selon la revendication 1, pour la préparation des dérivés de formule (I) pour lesquels $R_1$ = $CH_3$ ; X = méthylène ; n = 1 ou 2 ; $R_2$ = $R'_2$ = H ; $R_3$ = $CH_3$ ; et D représente oxygène.

6. Procédé selon la revendication 1, pour la préparation des dérivés de formule (I) pour lesquels $R_1$ = $CH_3$ ; X = méthylène ; n = 1 ou 2 ; $R_2$ = $R'_2$ = H ; $R_3$ = $CH_3$ ; et D représente N-OH(E).

7. Procédé selon la revendication 1, pour la préparation des dérivés de formule (I) pour lesquels $R_1$ = $CH_3$ ; X représente -CH = CH- ; n = 0 ou 1 ; $R_2$ = $R'_2$ = H ; $R_3$ = $CH_3$ ; et D représente oxygène.

8. Procédé selon la revendication 1, pour la préparation des dérivés de formule (I) pour lesquels $R_1$ = $CH_3$ ; X représente CH = CH ; n = 0 ou 1 ; $R_2$ = $R'_2$ = H ; $R_3$ = $CH_3$ ; et D représente N-OH(E).

9. Procédé selon la revendication 1, pour la préparation du dérivé de formule (I) pour lequel $R_1$ = $CH_3$ ; X = méthylène ; n = 1 ; $R_2$ = $R'_2$ = H ; $R_3$ = $CH_3$ ; et D représente oxygène.

10. Procédé selon l'une des revendications 3 à 9, pour la préparation des dérivés de formule (I) pour lesquels l'atome de carbone asymétrique est de configuration (R).

Office européen des brevets

# RAPPORT DE RECHERCHE EUROPEENNE

Numero de la demande

EP 90 40 2893

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | GB-A-2 076 813 (DELALANDE)<br>* Revendications 1,2,10,11 *<br>--- | 1,11-13 | C 07 D 263/24<br>A 61 K 31/42 |
| X | FR-A-2 458 547 (DELALANDE)<br>* Revendications 1-31; pages 14,15,43; composés 237,238 *<br>--- | 1,11-13 | |
| A | GB-A-2 003 151 (DELALANDE)<br>* Revendications *<br>----- | 1,11-13 | |

DOMAINES TECHNIQUES RECHERCHES (Int. Cl.5)

C 07 D 263/00

Le présent rapport a été établi pour toutes les revendications

| Lieu de la recherche | Date d'achèvement de la recherche | Examinateur |
|---|---|---|
| LA HAYE | 10-01-1991 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)